# EUROPEAN PATENT APPLICATION

(11) **EP 1 026 156 A2**
(43) Date of publication of application: **09.08.2000**
(21) Application number: 00300767.1
(22) Date of filing: 01.02.2000
(51) Int. Cl.: C07D 219/02, C07D 219/04, C07D 219/06, G01N 33/58, G01N 33/533

(54) **Novel 10, 10'-substituted-9,9'-biacridine luminescent molecules and their preparation**

(30) Priority: 01.02.1999 US 241513
(71) Applicant: PIERCE CHEMICAL COMPANY, Rockford, IL 61105 (US)
(72) Inventor: Katsilometes, George C., Idaho 83246 (US); Bremmer, Martin L., Rockford, Illinois 61111 (US); Hermanson, Greg T., Loves Park, Illinois 61073-8204 (US); Stack, Jeffrey G., Roscoe, Illinois 61073-8204 (US); Feather-Henigan, Kelli D., Rockford, Illinois 61108 (US); Hines, Kimberley, Crystal Lake, Illinois 60014 (US)
(74) Representative: Bowman, Paul Alan

(57) **Abstract**

Novel symmetrical and asymmetrical 10,10'-substituted-9,9'-biacridines and the synthesis of such symmetrical and asymmetrical 10,10'-substituted-9,9'-biacridine molecules and their derivatives is disclosed. These molecules are shown to produce light by chemiluminescence in the presence of signal solutions. These symmetrical or asymmetrical 10,10'-substituted-9,9'-biacridines are used alone or attached to haptens or macromolecules and are utilized as labels in the preparation of chemiluminescent, homogeneous or heterogeneous assays. They are also used in conjunction with other chemiluminescent label molecules to produce multiple analyte chemiluminescent assays.

## Description

The present invention relates to chemiluminescence, to chemiluminescent systems, and to novel compounds used in chemiluminescent systems and their methods of synthesis.

Luminescence is the emission of light without significant amounts of attendant heat. A specific light-emitting state is effected within a chemical without greatly increasing the temperature of the chemical. The light-emitting state is usually effected by providing energy to a molecule, generating the light-emitting state. The particular wavelength of radiation which is emitted by the molecule is determined by the characteristics of the molecule, and that wavelength does not usually change when the energy source to the molecule is changed.

Chemiluminescence is a special situation of luminescence, where the energy is directed to the molecule by a chemical reaction. Chemiluminescence involves substantially direct conversion of chemical energy to light energy. A number of different classes of compounds have been found to be particularly susceptible to chemiluminescent reactions. Exemplary classes of chemiluminescent compounds are phthalazinediones, especially the 2,3-dihydro-1,4-phthalazinediones (e.g., EPO 0 116 454 A2, T. H. Whitehead et al., 1985; and U.S. Patent No. 4,853,327, N. Dattagupta).

Measurement of light energy has become a useful method for monitoring the presence or concentration of substances in various media. Numerous bioluminescent and chemiluminescent reaction systems have been devised to implement this type of method (Schroeder et al., Methods in Enzymology, 17:24-462 (1978): Zeigler, M. M., and T. O. Baldwin, Current Topics In Bioenergetics, D. Rao Sanadi ed. (Academic Press) pp. 65-113 (1981); DeLuca, M., Non-Radiometric Assays: Technology and Application in Polypeptide and Steroid Hormone Detection, (Alan R. Liss, Inc.) pp. 47-60 and 61-77 (1988); DeJong, G. J., and P. J. M. Kwakman, I of Chromatography, 492:319-343 (1989); McCapra, F. et al., J. Biolumin. Chemilumin., 4:52-58 (1989); Diamandis, E. P., Clin. Biochem, 23:437-443 (1990); Gillevet, P. M., Nature, 348:657-658 (1990); Kricka, L. J., Amer Clin. Lab, Nov/Dec:30-32 (1990)).

Chemiluminescence is the emission of light only by means of a chemical reaction and may be further defined as the emission of light during a reversion to the ground state of electronically excited products of chemical reactions (Woodhead, J. S. et al., Complementary Immunoassays, W. P. Collins ed. (John Wiley & Sons Ltd.), pp. 181-191 (1988)). Chemiluminescent reactions are often discussed in terms of either enzyme-mediated or nonenzymatic reactions. It has been known for some time that the luminescent reactant luminol can be oxidized in neutral to alkaline conditions (pH 7.0-10.2) in the presence of: a) oxidoreductase enzymes (horseradish peroxidase, xanthine oxidase, glucose oxidase), b) H₂O₂, c) certain inorganic metal ion catalysts or molecules (iron, manganese, copper, zinc), and
d) chelating agents, and that this oxidation leads to the production of an excited intermediate (3-aminophthalic acid) which emits light on decay to its ground state (Schroeder, H. R. et al., Anal. Chem., 48:1933-1937 (1976); Simpson, J. S. A. et al., Nature, 279:646-647 (1979); Baret, A., U.S. Patent No. 4,933,276)). Other specific molecules and derivatives used to produce luminescence include cyclic diacyl hydrazides other than luminol (e.g., isoluminols), dioxetane derivatives, acridinium derivatives and peroxyoxylates (Messeri, G. et al., J. Biolum. Chemilum, 4:154-158 (1989); Schaap, A. P. et al., Tetrahedron Lett., 28:935-938 (1987); Givens. R. S. et al., ACS Symposium Series 383; Luminescence Applications, M. C. Goldberg ed. (Amer. Chem. Soc., Wash. D.C., pp. 127-154 (1989)). Additional molecules which produce light and have been utilized in the ultra sensitive measurement of molecules are polycyclic and reduced nitropolycyclic aromatic hydrocarbons, polycyclic aromatic amines, fluorescamine-labeled catecholamines, and other fluorescent derivatizing agents such as the coumarins, ninhydrins, o-phthalaldehydes, 7-fluoro-4-nitrobenz-2,1,3-oxadiazoles, naphthalene-2,3-dicarboxaldehydes, cyanobenz[f]isoindoles and dansyl chlorides (Simons, S. S., Jr. and D. F. Johnson, J. Am. Chem. Soc., 98:7098-7099 (1976); Roth, M., Anal Chem, 43:880-882 (1971); Dunges, W. *ibid*, 49:442-445 (1977); Hill, D. W. et al., *ibid*, 51:1338-1341 (1979); Lindroth, P. and K. Mopper, *ibid*, 51:1667-1674 (1979); Sigvardson, K. W. and J. W. Birks, *ibid*, 55:432-435 (1983); Sigvardson, K. W. et al., *ibid*, 56:1096-1102 (1984); de Montigny, P. et al., *ibid*, 59:1096-1101 (1987); Grayeski, M. L. and J. K. DeVasto, *ibid*, 59:1203-1206 (1987); Rubinstein, M. et al., Anal. Biochem., 95:117-121 (1979); Kobayashi, S.-I. et al., *ibid*, 112:99-104 (1981); Watanabe, Y. and K. Imai, *ibid*, 116:471-472 (1981); Tsuchiya, H., J. Chromatog, 231:247-254 (1982); DeJong, C. et al., *ibid*, 241:345-359 (1982); Miyaguchi, K. et al., *ibid*, 303:173-176 (1984); Sigvardson, K. W. and J. W. Birks, *ibid*, 316:507-518 (1984); Benson, J. R. and P. E. Hare, Proc. Nat Acad. Sci , 72:619-622 (1975); Kawasaki, T. et al., Biomed Chromatog., 4:113-118 (1990)).

There are currently four known nonenzymatic chemiluminescent systems: 1) the acridinium derivatives (McCapra et al., British Patent No. 1,461,877; Wolf-Rogers J. et al., J. Immunol Methods, 133:191-198 (1990)); 2) isoluminols, 3) metalloporphyrins (Forgione et al., U.S. Patent No. 4,375,972) and 4) nonmetallic tetrapyrroles (Katsilometes, PCT International Publication No. WO 93/23756 and U.S. Patent No. 5,340,714). These four non-enzymatic systems have certain advantages over the enzyme-mediated systems in that they tend to have faster kinetics resulting in peak light output within seconds. The rnetalloporphyrins are small hapten molecules which decrease steric hindrance problems in antigen binding. In addition, the metalloporphyrin molecules known to be luminescent are those containing a paramagnetic metal ion with emission yields above 10⁻⁴ (Gouterman, M., The Porphyrins, Vol. III, Dolphin, D., ed. (Academic Press): 48-50, 78-87, 115-117, 154-155 (1978); Canters, G. W. and J. H. Van Der Waals, *ibid*, 577-578). It is known that metalloporphyrins, hyposporphyrins, pseudonormal metalloporphyrins and metalloporphyrin-like molecules such as metallic chlorins, hemes, cytochromes, chlorophylls, lanthanides and actinides undergo oxidation/reduction reactions which are either primary or secondary to structural perturbations occurring in the metallic center of these molecules and that their reactive ability to catalyze the production of chemiluminescence has been ascribed to the metallo center of these molecules (Eastwood, D. and M. Gouterman, J. Mol. Spectros, 35:359-375 (1970); Fleischer, E. B. and M. Krishnamurthy, Annals N.Y Academy of Sci., 206:32-47 (1973); Dolphin. D. et al., *ibid*, 206:177-201; Tsutsui, M. and T. S. Srivastava, *ibid*, 206:404-408; Kadish, K. M. and D. G. Davis, *ibid*, 206:495-504; Felton, R. H. et al., *ibid*, 206:504-516; Whitten, D. G. et al., *ibid*, 206:516-533; Wasser, P. K. W. and J.-H. Fuhrhop, *ibid*, 206:533-549; Forgione et al., U.S. Patent No. 4,375,972; Reszka, K. and R. C. Sealy, Photochemistry and Photobiology, 39:293-299 (1984); Gonsalves, A.M.d'A. R. et al., Tetrahedron Lett, 32:1355-1358 (1991)). These reactions are altered by iron and other metal ions which may be present in the reactants and these metal ions can interfere with and greatly confound the assay of metalloporphyrin conjugate concentrations (Ewetz, L. and A. Thore, Anal. Biochem, 71:564-570 (1976)). Different metals will strongly influence the lifetimes and luminescent properties of the metalloporphyrins.

The nonmetallic porphyrin deuteroporphyrin-IX HC1 has been shown to luminesce and to mediate the production of light from luminol in solution (Katsilometes, G. W., *supra*).

Use of the luminescent acridinium ester and amide derivatives in chemiluminescent reactions and in the development of nonisotopic ligand binding assays has been reported and reviewed (Weeks, I. et al., Clin. Chem., 29/8:1474-1479 (1983); Weeks, I. and J. S. Woodhead, Trends in Anal Chem., 7/2:55-58 (1988)). The very short lived emission of photons (< 5 sec) to produce the flash-type kinetics in the presence of H₂O₂ and NaOH oxidation reagents (pH 13.0) is characteristic of the system.

Methods of preparation of acridones and variously substituted acridines and acridones have been summarized (Acridines, Acheson, R. M. and L. E. Orgel (Interscience Publishers, N.Y.) pp. 8-33, 60-67, 76-95, 105-123, 148-173, 188-199, 224-233 (1956)). Formation of biacridines by the combining of two acridine residues at the carbon-9 atom has been described and reviewed previously (Gleu, K. and R. Schaarschmidt, Berichte, 8:909-915 (1940)). These efforts led to the synthesis of 10,10'-dimethyl, 10,10'-diphenyl and 10,10'-diethyl-9,9'-biacridinium nitrate molecules. It was also reported that these molecules will produce light when exposed to hydrogen peroxide in basic solution (Gleu, K. and W. Petsch, Angew Chem., 48:57-59 (1935); Gleu, K. and R. Schaarschmidt, Berichte, 8:909-915 (1940)).

The mechanism of light production by lucigenin (10,10'-dimethyl-9,9'-biacridinium nitrate) has been extensively studied and has been ascribed to a series of hydroxide ion nucleophilic additions to acridinium salts and their reduction products (pinacols), culminated by the oxidation of the main end product N-methylacridone (Janzen, E. G. et al., J. Organic Chem., 35:88-95 (1970); Maeda, K. et al., Bul. Chem. Soc Japan, 50:473-481 (1977); Maskiewicz, R. et al., J. Am Chem Soc., 101/18:5347-5354 (1979); Maskiewicz, R. et al., *ibid*, 101/18:5355-5364 (1979)).

Modifications and derivatizations of 10-methyl acridine at the carbon-9 atom have led to the production of several useful chemiluminescent molecules having varying degrees of stability (Law, S.-J. et al., J. Biolum. Chemilum., 4:88-98 (1989)). These molecules produce a flash of light lasting less than five seconds when exposed to 0.5% w/v hydrogen peroxide in 0.1 mol/L nitric acid followed by a separate solution containing 0.25 mol/L sodium hydroxide.

A luminescent derivative, a luminescent derivatized molecule or a derivatized luminescent molecule as defined herein is a molecule which results from the covalent binding of a functional group or a group which changes the chemical reactivity and properties of a precursor molecule leading to the formation of a luminescent molecule suitable for conjugation to an analyte or a particular binding partner one wishes to use in assay development. A N-hydroxy succinimide derivatization of biacridines at one or both of the two 10,10' positions are the preferred luminescent derivatives of the invention. A compound or a molecule is a "derivative" of a first compound or first molecule if the derivative compound or molecule is formed (or can be formed) by reaction of the first compound or first molecule to form a new compound or new molecule either smaller or larger than the first compound or first molecule while retaining at least part of the structure of the first compound or first molecule. As used herein the term "derivative" can also include a "luminescent derivative".

Prior to the invention described in U.S. patent Applications Serial Nos. 08/265,481 (filed June 24, 1994), PCT Application WO 96/00392 (published April 1, 1996), and 08/767,288 (filed December 16, 1996), synthesis of derivatized luminescent 10,10'-substituted-9,9'-biacridines had not been achieved. The previously known luminescent biacridines (e.g., lucigenin) contained no reactive group(s) which permitted the conjugation of the biacridine molecule to another. Until then, the biacridines had academic interest only and were used to study the mechanism of light production and the interactions of reactive ionic species.

The use of luminescent reactions at the surface of light conductive materials (e.g., fiber-optic bundle) is the basis of the development of luminescent sensors or probes (Blum, L. J. et al., Anal Lett., 21:717-726 (1988)). This luminescence may be modulated by specific protein binding (antibody) and can be produced in a micro environment at the surface of the probe. The light output is then measured by photon measuring devices in the formulation of homogeneous (separation free) assays (Messeri, G. et al., Clin Chem, 30:653-657 (1984); Sutherland, R. M. et al., Complementary, Immunoassays, Collins, W. P., ed. (John Wiley & Sons, Ltd.) pp. 241-261 1988)).

It would be beneficial in improving assay sensitivity to increase the output of light obtained from chemiluminescent reactions by synthesizing biacridine molecules capable of producing superior quantities of photo emissions and by improving existing signal solutions and to have novel signal solutions which provide a greater intensity of light during chemiluminescent reactions. The ability to modulate the kinetics of light output through manipulation of the signal solution formula is particularly beneficial in tailoring assays for a variety of uses (genetic probe, sensor, hormones, etc.).

The present invention relates to novel 10,10'-substituted-9,9'-biacridinium compounds and the synthesis of these new 10,10'-substituted-9,9'-biacridinium compounds, the preparation of novel chemical solutions for the production of light from these new molecules, and the use of these new molecules in luminescent reactions and assays. More particularly, the invention describes the synthesis of symmetrical, uniformly symmetrical, and asymmetrical 10,10' -para-substituted-9,9'-biacridines, particularly 10-paratoluic acid (and derivatives, such as ester derivatives), 10'-para-substituted-9,9'-biacridines and the demonstration of the ability to covalently bind (conjugate) these new molecules to another molecule such as an antibody, hapten, streptavidin, biotin, nucleic acid, conjugated to a protein, carbohydrates, DNA, RNA, nucleosides, lipids, organic molecule, or organic polymer wherein the substituent substituted at the 10 position is different from the substituent substituted at the 10'position or any other appropriate molecule (particulalry biological molecules) for which an assay or detection is desired and to produce measurable light from bound chemiluminescent label molecule. The invention further involves luminescent signal solutions comprising at least one oxidant, sulfoxide, chelating agent, reducing sugar, and alcohol (as well as optional, alternative and/or preferable materials such as a buffer) in aqueous solution of oxidizing agent (such as sodium tetraborate, peroxides, hyperoxides, and superoxides) to produce high yield photon emissions useful in chemical assays, nucleic acid assays and immunoassays.

The terminology used in the practice of the present invention with regard to symmetry, uniform symmetry and asymmetry on these 10,10'-para-substituted-9,9'-biacridininium compounds has a specific meaning as used in the practice of the present invention, that is not inconsistent with conventional use of the terms. The terms "symmetrical" and "symmetry" as used in the present invention means that the actual groups on the 10-position and the 10'-position of the compounds are identical, irrespective of any other substitution on the 10,10'-para-substituted-9,9'-biacridininum compounds. That is, if the 10-position and the 10'-position are substituted with the identical group (e.g., p-toluic acid), the compounds are within the definition of symmetrical, whether or not there are different substituents on the remaining ring portions of the acridinium molecules. If there are identical substituents on the 10-position and the 10'-position of the biacridinium compound and the remainder of the positions on the acridinium moieties have identical substitution thereon, the compounds would be referred to as "uniformly symmetrical" in the practice of the present invention. Thus, a compound such as 10,10'-para-toluic acid-9,9'-biacridinium dinitrate is uniformly symmetrical, but a compound 10,10'-para-toluic acid-2-fluoro-9,9'-biacridinium dinitrate is within the definition of symmetrical (as would be the 10,10'-para-toluic acid-9,9'-biacridinium dinitrate) because both the 10 and the 10' positions have the same substituent. A compound such as 10-para-toluic acid-10'-methyl-biacridinium nitrate would not be symmetrical and would be classified as asymmetrical according to the present invention. It is also to be noted that throughout this text the terms biacridine and biacridinium salt are used. The term "biacridine" is often used as generic to the neutral organic compound and the salt (unless the term is used as a direct description of a compound named or shown by formula) and the term biacridinium salt must apply to only the salt form of the biacridine. This use of nomenclature is solely for purposes of convenience and is not an attempt to differentiate the invention between neutral compounds and salts.

The use of nucleophile-reactive substituent groups is desirable on either the symmetrical, uniformly symmetrical or asymmetrical biacridines used in the practice of the present invention. Non-limiting examples of nucleophile-reactive groups which may be present on symmetrical, uniformly symmetrical or asymmetrical biacridines include N-hydroxysuccinimide ester groups, sulfo-N-hydroxysuccinimide ester groups, polyfluorophenyl ester groups, acylimidazole groups, succinimidyl carbonate groups, carbonates, anhydride groups, hydrazides, acyl azides, haloacetyl groups, maleimide groups, pyridyl disulfide groups, epoxy functional groups, vinyl sulfone groups, aldehyde groups, isocyanate groups, isothiocyanate groups, phosphoramidine groups, and sulfonyl chloride groups. These nucleophile-reactive functionalities may be provided in any manner that leaves the reactive group available, as by using intermediate linking groups or by using di-functional materials to provide the nucleophile-reactive groups onto the biacridine moities. The placement of the nucleophile-reactive groups may be varied amongst any of the available positions on the acridine as is convenient according to available reactive mechanisms. In general, any of the groups and substituents described above may be provided by those of ordinary skill in the art by selection of the appropriate reagant and using known synthetic procedures.

The biacridines may also be substituted with groups that have photoresponsive characteristics (other than just photoreactive capability, as with the epoxy resin groups) such as UV sensitivity (which may of course be further spectrally sensitized by the use of sensitizing dyes) which may be provided by aryl azides, halogenated aryl azides, triazines and s-triazines, anthraquinones, benzophenones, psoralens, diazonium compounds (including positive-activing diazonium oxides), sulfonium salts, iodonium salts, and diazirine groups.

The classification of compounds within the terminology of symmetrical or asymmetrical according to the present invention is not affected by restricted rotation giving rise to perpendicular disymmetric planes, as where the compounds have chiral centers and form mirror image orientations. Even though these structures can be graphically or schematically represented such that no possible perpendicular planes can be drawn which can be bisected by a plane as visual symmetry, the term symmetry as used herein applies to chemical substitution only, not to mere physical orientation or mere optical activity and orientation. For example, the compounds of this invention may include "mono-substituted" compounds wherein only one of the 10 or 10' hydrogens in a biacridine is replaced to form a mono-substituted biacridine or monosubstituted biacridium salt.

One aspect of the invention is the method for detecting the presence of the novel biacridine luminescent compounds in a sample. The method comprises contacting the sample with a signal solution to produce, by means of chemiluminescence, measurable emitted light and measuring the emitted light with a photometric instrument or device.

Another aspect of this invention is methods for the synthesis of luminescent, symmetric, uniformly symmetrical and asymmetric 10,10'-substituted-9,9'-biacridine molecules which can be bound to analyte or to a binding partner of analyte or to a ligand of a binding partner to analyte. These molecules may have additional substitutions at other sites on the molecule such as carbon atoms 1 through 8 on the acridone precursor by selection of the appropriate reagent (e.g., the appropriately substituted acridone). Preferred substituents on the 1 through 8 carbon atoms of the biacridinium compounds may be selected from the group consisting of alkyl groups, alkenyl groups, amino groups, carbonyl groups, hydroxy, and aryl groups (i.e., the term groups meaning substituted or not), with halogenated alkyl (especially perfluorinated alkyl groups) particularly contemplated, halogen (fluorine, bromine, chlorine and iodine), sulfonate, alkoxy, aryloxy, carboxyl, nitrile, inorganic acids groups (e.g., sulfonic acid, phosphonic acid), hetero atoms as bridging groups (e.g., sulfur, nitrogen, oxygen, boron), and the like. As an option within practice of the present invention, the substitution (on any one of R1, R2, R3, R4, R5, R6, R7, R8, R9 or R10, individually, collectively or in any combinations and permutations thereof) should not be or way not be amino or substituted amino. In Campbell et al., U.S. Patent No. 4,478,817, column 5-6, it is described that where the molecule is symmetrical at the 10 and 10' positions, the molecule must be trisubstituted with the same moiety; Campbell at al. do not depict or describe asymmetrical biacridines as defined in the practice of this invention. Campbell et al. Does not show tetra- or penta- substituted compounds (e.g., where R5 and R6 are the same moiety and those moities appear as substitutents on two (Tetra) or three (penta) othre positions on the biacridine or biacridium molecule.

Still another aspect of the invention is directed to a chemiluminescent system for emitting measurable light useful in a chemical assay, a ligand binding assay, an immunoassay or a nucleotide assay. The system comprises, at a pH ranging from about 10.0 to about 14.0, an uniformly symmetrical, symmetrical or asymmetrical 10,10'-substituted-9,9'-biacridine with a specific energy of activation and oxidation potential, bound to analyte, or to a binding partner of analyte or to a ligand of a binding partner to analyte and an oxidant or a combination of oxidants capable of overcoming the inherent oxidation potential of the biacridine. In this system the biacridine acts as a luminescent label (tag) for the production of chemiluminescence in chemical assays, homogeneous, heterogeneous competitive and sandwich immunoassays, ligand binding assays and nucleotide assays. The light is produced upon exposure of the biacridine label to signal solution having the nucleophilic reactants and oxidant or oxidants.

The uniformly symmetrical, symmetrical and asymmetrical 10,10'-substituted-9,9'-biacridines are especially beneficial as the label is more sensitive (i.e., detects smaller quantities of analyte) than known chemiluminescent labels and are able to undergo modification of the kinetics of light production though manipulation of the signal solution formula resulting in light production for at least 0.1 second and also for as long as 6 seconds or longer.

A further aspect of the invention is a method for the synthesis of novel uniformly symmetrical, asymmetrical and symmetrical 10,10'-substituted-9,9'-biacridines which have luminescent properties. Theie methods involves the alkylation of the starting material (acridone or selectively substituted acridone) to form the corresponding N-substituted acridone followed by dimerization of this product (e.g., using a catalyst and acid, such as a metal based catalyst [e.g., zinc] and inorganic acid such as hydrochloric acid). Oxidation of the resulting acrylidine is accomplished with an oxidizing acidic environment, preferably with an oxidizing acid, such as nitric acid, to yield the 10,10'-substituted-9,9'-biacridinium di(anion), e.g., dinitrate. If necessary, the biacridinium salt may be derivatized by reaction with, for example, N-hydroxysuccinimide and dicyclohexylcarbodiimide to give the corresponding N-hydroxysuccinimide (NHS) ester.

Another aspect of the invention is chemiluminescent signal solutions which when reacted with chemiluminescent label that is a luminescent molecule produces chemiluminescence. The signal solution comprises, for example, at a pH from about 10.0 to about 14.0, 0.02 M sodium tetraborate (borax), ethylenediaminetetra-acetic acid (EDTA), dimethyl sulfoxide (DMSO), D(-) fructose, potassium superoxide (KO₂) and 2-methyl-2-propanol. Where the chemiluminescent label is an acridinium derivative, lucigenin, a lucigenin luminescent derivative, a biacridine, a biacridinium luminescent derivative, a cyclic diacyl hydrazide or a pteridine, the reaction will produce a signal-to-noise photon emission ratio of at least 20:1 at 1 ng/ml of label concentration for at least 0.1 seconds duration. Depending upon the label and the variation of the concentration of the signal solution components, the signal ratio can be 50:1, 100:1, 200:1, 500:1, and even 700:1 and greater at 1 ng/ml of label concentration. The emission can also he manipulated to last up to 6 seconds or longer. Additional oxidants, without this list attempting to be limiting in the practice of the present invention, includes: urea/H₂O₂ complex, oxone, potassium or ammonium persulfate, hydroperoxides, peracids (peracetic, m-CPBA), ceric ammonium nitrate, hypochlorites, periodic acid, periodates such as Na periodate, KMnO₄, amine oxides, dioxitanes, Na percarbonate, percarbonates, peracids such as perbenzoic acid, H₂O₂/hexafluoroacetone adduct, and inorganic or organic peroxides.
Figure 1 shows a graph of the Relative Chemiluminescence of solutions as Assay Condition versus Counts per Second.
Figure 2 shows the intensity of light output from a biacridine sysem.
Figure 3 shows a graph of Relative Chemiluminescence of an Antibody-Biacridine Conjugate in Counts per Second.
Figures 4A and 4B show graphs of the light emission kinetics for the signal solution described in Example 2.

As defined herein, a signal solution comprises a reagent or group of reagents which, when combined with a specific luminescent molecule or a specific luminescence mediating molecule, will cause the production of light. A luminescent label or tag as described herein is a substance bound to analyte, a binding partner of analyte, or to a ligand of a binding partner of analyte either directly (e.g., covalently) or indirectly (e.g., by means of a specific binding substance (e.g., antibody, protein, etc.), a biotin-avidin or biotin-streptavidin bridge) which when combined with a signal solution either produces light or causes light to be produced. A luminescent label is a luminescent molecule (i.e., the substance which emits light).

As defined herein, a luminescent molecule is a substance, which following light, electrical, chemical and/or electronic excitation (e.g., by constituents of a chemical solution), will emit a photon(s) upon decay of orbital electrons to ground state.

As used herein, a luminescent reactant is a free luminescent molecule (i.e., a luminescent molecule that is not bound to analyte, a binding partner of analyte or to a ligand of a binding partner of the analyte). Also as used herein, the singular term "luminescent molecule" can also include the plural "luminescent molecules". Also as used herein, the singular term "luminescence mediating molecule" can also include the plural.

The term asymmetric or asymmetrical, unless otherwise stated, requires only that different substitution exist on the 10 and 10' positions. The same or different substituents may be present on the various other substitutable positions on the biacridine (e.g., on the fused benzene rings), but asymmetry must exist as between the 10 and 10' positions, unless otherwise specifically stated when the term asymmetrical or asymmetrical is used.

The term group, as applied to a class of chemical compounds, e.g., alkyl group, refers to not only the literal class (e.g., methyl, ethyl, hexyl, cyclohexyl, iso-octyl, dodecyl, etc.), but also to the substituted counterparts of the class (e.g., hydroxymethyl, 3-chloropropyl, 1,1,1-trifluorohexyl, and the like). Where the terms moiety, species or no qualification are used (e.g., alkyl, alkyl moiety, or alkyl species), the term includes only the literal class, without substitution thereon. Typical substituents which may be used on alkyl and aryl (e.g., phenyl) groups within the practice of the present invention may include, without intending to limit the scope of the invention, alkyl (straight-chain, branched chain, and cycloalkyl) groups, alkoxy groups, halogen (e.g.. F, Cl, Br, and I), hydroxy, carboxylic acid and carboxylate, acetyl, nitro, amino (except as restricted, limited or excluded herein by specific definitions), sulfonate, phosphate, esters, ethers (including thioethers), and other conventional substitution as known within the art. The counterion (e.g., the anion, typically shown as nitrate) similarly may be varied as well understood within the art. Halide counterions (e.g., chloride, fluoride, bromide and iodide) are useful as is any other counterion, whether simple or complex, again exemplified by the following non-limiting examples of nitrate, nitrite, paratoluene sulfonate, sulfate, phosphate, carbonate, PECHS (perfluoroethylenecyclohexylsulfonate), and especially other acid anions.

The invention is also directed to methods for synthesizing asymmetric, uniformly symmetric and symmetric luminescent 10,10'-substituted-9,9'-biacridine derivative molecules which can be bound to analyte, a binding partner of analyte, or to a ligand of a binding partner of analyte either directly or indirectly.

The nature of the symmetrical, uniformly symmetrical or asymmetric 10,10'-substituted-9,9'-biacridine compounds and systems of the present invention may be better appreciated by reference to the formulae for the compounds of the present invention. Formula I, for example, shows a general formula for 10,10'-substituted-9,9'-biacridine compounds having R5 and R6 10 and 10' substituents and R1, R2, R3 and R4 substituents on the non-nitrogen positions (positions 1, 2, 3, 4, 5, 6, 7 and 8, with four positions on each of the fused benzene rings on the central nucleus of the acridines) of the acridine nuclei, and two anions X⁻. The compounds are symmetrical according to the present invention when R5 and R6 are identical, irrespective of the nature of groups R1, R2, R3, R4, R7 and R8, and even if the rings are multiply substituted. It is to be noted that there may be independently more than one of R1, R2, R3 and R4 on the rings, which may be alternatively named R7, R8, R9 and R10 on the structurakl formula. It is an option that the substitution on the fused benzene rings be limited to only one R1, R2, R3 or R4 group, but up to four of each group may be present. Where R1, R2, R3, R4, R7 and R8 are the same on parallel or symmeytric locations (including hydrogen) and R5 and R6 and identical with each other, the compound is completely symmetrical or "uniformly symmetrical" as defined in the practice of the present invention. For example, the 10,10'-substituted-9,9'-biacridines of formulae II, III, IV, V, VI, VII, VIII, IX, X, XVIII, and XIX are uniformly symmetrical, while 10,10'-substituted-9,9'-biacridine compounds XIII, XIV, XV, XVI, and XVII are asymmetrical. To be asymmetrical, R5 and R6 must be different, irrespective of the symmetry or asymmetry of the other substituent groups. These asymmetrical compounds may be most easily formed in mixtures or solutions with symmetrical compounds by reacting two differently substituted acridones (e.g., Formulae XI and XII) together to get a mixture of dimethyl-substituted, di-toluic acid substituted, and monomethyl-monotoluic acid substituted 10,10'-substituted-9,9'-biacridine. They may be separated or purified (as by high performance liquid chromatography). They can also be prepared by other processes and procedures referred to herein, such as leaching, separation by differentiating solubility, and the like.

Substitution may also be used to adjust the physical properties of the biacridine such as its relative hydrophilicty or hydrophobicity. This can be readily acomplished by the addition of groups onto the biacridine which may not be active with regard to the chemiluminescent performance of the biacridine but adjust its compatability to the solutions or environment in which it is intended to be used. For example, one may attach pendant or spacing units that are hydrophilic or hydrophobic by virtue of charged groups or substituents polar constituents, as by the presence or addition of polyoxyalkylene (especially polyoxyethylene) bridging groups, polyethyleneglycol-type bridging groups or pendant groups, hydrocarbon compounds, organic compounds containing secondary amine groups (e.g., terminal, pendant or spaced periodically) such as with triethylenetetramine, spermine or pentaethylenehexamine, the addition of pendant hydrophilic groups such as sulfonate groups, sulfonamide groups, carboxy groups, phosphoric acid groups, phosphonic acid groups and the like. For example, a taurine group may be incorporated at one of the 10 or 10' positions, or a sulfo-N-hydroxysuccinimide group may be incorporated as an amine-reactive component. Reactive groups described herein may be built off of one or more of these hydrophilic spacer groups, resulting in greater conjugate stability alter reaction with another molecule. This strategy would help to overcome the hydrophobicity of the biacridne molecule, thus decreasing the potential for precipitation after a molecule has been modified. An asymmetrical biacridine containing a charged taurine group, for example, on one of the 10 or 10' positions and a reactive group constructed from the use of a hydrophilic spacer on the other position constitues a particularly useful modification reagent that has greatly enhanced water solubility.

The symmetrical, uniformly symmetrical and asymmetrical biacridines described herein may also be used to measure or detect superoxide in solution or in cellular environments. A variety of methods have been developed to detect or assay superoxide (O₂⁻) in solution or in a cellular environment. Transient superoxide in solution gives a UV absorbancy with a peak at 240 nm (extenction coeeficient is about 1060). Other spectrophotometric analysis is possible as well, including infrared, Raman, and superoxide analysis. More accurate testing for superoxide, however, relies upon its redox properties creating or altering the spectral characteristics of other molecules. For example, reduction of tetranitromethane to nitroform anions yields an absorbance at 350 nanometers and an extinction coefficient of about 15,000. Another common test involves the reduction of nitrotetrazolium blue to a diformazan (with a wavelength of maximum absorption at 560 nm and an extinction coefficient of about 10,000). Similarly, reduction of ferricytochrome derivatives to ferricytochromes can be monitored at 550 nanometers.

Oxidative reactions can also be used to detect the presence of superoxide. Oxidation of epinephrine to adrenochrome yields and absorbance at 480 nm. Perhaps the most common oxidative method involves the chemiluminescence of certain organic compounds. Lucigenin (bis-N-methylacridinium dinitrate) frequently has been employed to monitor superoxide production by cells. Superoxide reacts with lucigenin to produce an unstable dioxetane which decomposes to give electronically excited N-methyl acridone whose return to the ground state yields a photon of light.

The symmetrical, uniformly symmetrical or asymmetrical biacridine molecules of the present invention may be valuable new tools for measuring superoxide in solution or in cellular environmnets. In particular, we have found that a superoxide triggering solution provides a very intense flash of light with the new biacridine derivative compounds. The biacridine compounds described herein for the first time may provide a mechanism for following superoxide production within cells. Targeting molecules such as antibodies labeled with one of the biacridine compounds of the present invention might be used to bind to important superoxide signaling proteins within cells, and to monitor superoxide synthesis by chemiluminescence.

The invention is also directed to a method for detecting in a sample the presence of a symmetric, uniformly symmetric or asymmetric 10,10'-substituted-9,9'-biacridine or biacridinium salt luminescent derivative having a specific energy of activation and oxidation potential. The method comprises contacting the sample with a signal solution which comprises at least one oxidant capable of overcoming the specific energy of activation or oxidation potential of the symmetric, uniformly symmetric or asymmetric 10,10'-substituted-9,9'-biacridine. The light emission may occur (without being limited to this theory) because of the formation and decomposition of a high energy dioxetane intermediate, with the actual light emission resulting from a change in the electronic state of the resulting acridone. The symmetric, uniformly symmetric or asymmetric 10,10'-substituted-9,9'-biacridine and the oxidant react to produce emitted light by means of chemiluminescence. Postulated mechanisms for the chemiluminescent production of light by symmetric or asymmetric 10,10'-substituted-9,9'-biacridines would begin with the addition of nucleophiles such as hydroxide ions to the biacridine leading to schism of the dimer between 9,9'-carbon atoms. This schism produces two excited state N-methylacridone molecules which produce light as discussed by Janzen, et al., Maeda, et al., and Maskiewicz, et al., *supra*. It is also hypothesized that abstraction of an electron from an appropriate luminescent molecule leads to the formation of the excited intermediate which emits a photon upon decay of the luminescent molecule to the ground energy state. The light is then measured preferably with a photometric instrument or device such as the Berthold Lumat LB 950 luminometer. This method is more sensitive and more accurate due to lack of interference and self absorption problems encountered with the usual fluorometric methods used to detect fluorophores in solution.

Potassium superoxide is preferred for overcoming the oxidation potential of the 10,10'-substituted-9,9'-biacridine. However, other oxidants or potassium superoxide in conjunction with other oxidants such as osmium tetroxide or hydrogen peroxide, is also another oxidant mixture capable of overcoming the inherent oxidation potential of the 10,10'-substituted-9,9'-biacridine.

The invention is also directed to a chemiluminescent system for emitting measurable light useful in chemical assays, in ligand binding assays such as an immunoassays or in nucleotide assays. This system comprises at a pH ranging from about 10.0 to about 14.0, an asymmetric, uniformly symmetric or symmetric 10,10'-substituted-9,9'-biacridine luminescent derivative having an oxidation potential bound to analyte or to a binding partner of analyte or to a ligand of a binding partner to an analyte, and at least one oxidant which is/are capable of lowering the oxidation potential of the 10,10'-substituted-9,9'-biacridine. Essentially the 10,10'-substituted-9,9'-biacridine acts as a label (i.e., a tag or tracer) and produces light in the chemiluminescent reaction. The immunoassay may be homogeneous or heterogeneous and a competitive or sandwich assay. The light is produced by the 10,10'-substituted-9,9'-biacridine by means of chemiluminescence upon exposure of the 10,10'-substituted-9,9'-biacridine to an oxidant or oxidants.

This system is particularly useful for detecting an analyte such as a nucleic acid, an antibody, an antigen, a hapten or hapten conjugate, a macromolecule, a protein or a polymer. A binding partner to an analyte in this system may be a nucleotide probe, an antibody, an antigen, DNA. RNA, a hapten, a hapten conjugate, a macromolecule, a protein or a polymer.

A ligand used herein means a linking or binding molecule and may include antigen, antibody, hapten, hapten conjugate, macromolecule, protein or polymer other than a protein such as a polyhydrocarbon, a polyglyceride or a polysaccharide.

A hapten conjugate as used herein is a small molecule (i.e., a molecule having a molecular weight of less than 6,000 Daltons) that is a attached to another molecule. An example of a particularly suitable hapten conjugate is a steroid molecule-10,10'-substituted-9,9'-biacridine conjugate. The analyte may be bound to the binding partner or the binding partner may be bound to the ligand by means of a biotin-avidin or a biotin-streptavidin bridge. The ligand may also be biotin, avidin or streptavidin and the analyte may also be bound to the 10,10'-substituted-9,9'-biacridine by means of the biotin-avidin, biotin-streptavidin system. The system provides great sensitivity (up to 10⁻¹⁶ to 10⁻²⁰ molar detection of antibody or antigen) when the system comprises 10,10'-substituted-9,9'-biacridine luminescent label and potassium superoxide as the oxidant. An even greater sensitivity (up to 10⁻²² molar detection of antibody or antigen) is obtained when the system comprises a chelating agent DMSO, D(-) fructose, 2-methyl-2-propanol, aqueous sodium tetraborate and an oxidant or combination of oxidants capable of overcoming the oxidation potential.

While the chemiluminescent system will be effective at a pH ranging from about 10 to about 14, the preferred pH is from a pH of about 12.5 to 13.5.

The chemiluminescent properties of the 10,10'-substituted-9,9'-biacridine tag together with the other reagents in the system make the system particularly suitable for the development of ultrasensitive assays for many hapten and macromolecular analytes to which the 10,10'-substituted-9,9'-biacridine can be directly or indirectly conjugated such as hormones, vitamins, toxins, proteins, infectious and contagious agents, chemicals, drugs, tumor markers, receptors, biotin, avidin, streptavidin and genetic material. The 10,10'-substituted-9,9'-biacridine can also be directly or indirectly conjugated to a specific binding protein such as an antibody for use in chemiluminometric assay development.

The invention is further directed to a chemiluminescent system for emitting measurable light useful in a chemical assay, an immunoassay, a ligand binding assay or a nucleic acid assay which comprises a 10,10'-substituted-9,9'-biacridine having a specific oxidation potential, bound to an analyte, or to a binding partner of an analyte or to a ligand to a binding partner to an analyte and a signal solution which comprises, at a pH ranging from about 10.0 to about 14.0, the oxidant, potassium superoxide, or a combination of oxidants comprising osmium tetroxide and potassium superoxide.

Examples of luminescent molecules for use with the signal solution in this invention are the acridinium derivatives, pteridines, pteridine derivatives, lucigenin, lucigenin derivatives, luciferin, luciferin derivatives, cyclic diacyl hydrazides (e.g., luminol or isoluminol), an acridinium derivative such as dimethyl acridinium ester or luciferin and lucigenin derivatives such as those resulting from N-hydroxy succinimide derivatizations are preferred.

Again, this chemiluminescent system lends itself to heterogeneous and homogeneous assays including competitive and sandwich immunoassays. The sensitivity of the system is extremely high when the signal solution comprises the EDTA, DMSO, D(-) fructose, at least one oxidant, 2-Methyl-2-propanol and aqueous sodium tetraborate as described above. Again, the analyte may be nucleic acid, antigen, antibody, hapten, hapten conjugate, macromolecule, protein or polymer. It has been speculated that the homogeneous assay, under certain unique environmental circumstances, might involve the use of inhibitors of label luminescence such as polyions. A polycation such as poly(4-vinylpyridinium dichromate) would inhibit, for example, an unbound deuteroporphyrin IX dihydrochloride (DPIX) labeled compound while a polyanion such as poly(vinylalkyl) would inhibit an unbound positively charged 10,10'-substituted-9,9'-biacridinium labeled compound. Unbound in this instance of an assay means that if, for example, the compound is an antigen-label conjugate, it is not bound to, for example, an antibody or if the compound is an antibody-label conjugate it is not bound to an antigen, etc.

The invention is also directed to a method for using symmetrical, uniformly symmetrical or asymmetrical 10,10'-substituted-9,9'-biacridine in a chemiluminescent heterogeneous assay for detecting the presence of multiple analytes in a sample. Suitable analytes for detection are nucleic acids, antibodies, antigens, haptens, hapten conjugates, macromolecules, polymers or proteins. Again, the method can be a chemical assay, a nucleotide, assay or a ligand binding assay such as an immunoassay. The method may also be a combination of any of these assays. The invention involves the conjugation of 10,10'-substituted-9,9'-biacridine tag to a first analyte or to a binding partner of that analyte or to a ligand of a binding partner of that analyte and the binding of a different tag(s) or label(s) such as a nonmetallic tetrapyrrole luminescent molecule or a molecule that mediates chemiluminescence such as an enzyme to other (e.g., a second) analyte or to a binding partner of the other analyte or to a ligand of the binding partner of the other analyte. The analytes may be polynucleotide strand, chemically active compound such as chlorine or tetrapyrroles, or immunologically active compound such as antibody, antigen, hapten, hapten conjugate, macromolecule, protein or polymer.

Generally, in the multiple sandwich-type immunoassay, a binding partner to one site on the first analyte is attached to a solid phase such as glass, polypropylene, polycarbonate or polystyrene and the, thus, coated solid phase is contacted with the sample and other binding partner for an other (e.g., second) site on the analyte. The other binding partner is conjugated to the label (e.g., the 10,10'-substituted-9,9'-biacridine derivative). The same situation exists for the other or second analytes only the label(s) and the binding partner(s) are naturally different. The solid phase is washed and the bound conjugates are exposed to the appropriate signal solution or signal solutions.

Generally, in a competitive assay, the solid phase is coated with limited concentrations of binding partners specific for each analyte of interest. The solid phase is then contacted with the sample and with a measured amount of first analyte conjugated to the 10,10'-substituted-9,9'-biacridine and with a measured amount of other analyte(s) conjugated to the other luminescent label(s). Following contact, the solid phase is washed to remove any unbound conjugate. With both the sandwich-type or competitive-type assay, the washed solid phase may be separately treated first with a signal solution specific for only one of the two or more labels wherein the label and the solution react to produce emitted light and the amount of analyte related to that specific label may be determined by measuring the amount of light emitted, the solid phase can then be separately contacted with other chemiluminescent signal solution specific for the other label(s) or tag(s) relating to the other analyte(s) whereby those label(s) and signal solution(s) react to produce emitted light. Again, the measurement of the light from the other (e.g., second) reaction(s) will determine the amount of other analyte(s) present in the sample.

Since the light produced as a result of the two different labels has different properties (i.e., the wavelength of light given off by means of each label may differ or the actual amount of light produced per second of reaction may differ between the two labels), it is possible to treat the solid or liquid (e.g., washed) phase with a signal solution which will produce light by multiple conjugates simultaneously, differentiate that light and measure the light to determine the amount of each analyte in the sample. One can differentiate the light given off as a result of the different labels by utilizing time resolved luminescent analysis such as that used in fluorometry (Lovgren, T. and K. Pettersson, Luminescence Immunoassay and Molecular Applications, Van Dyke, K. and R. Van Dyke eds., CRC Ress, Boca Raton, Ann Arbor, Boston, MA, pp. 233-254 (1990)).

The differences in emission properties such as wavelengths can also be utilized (Kleinerman, M. et al., Luminescence of Organic and Inorganic Materials, Kallmann, H. P. and G. M. Spruch eds., International Conference, New York University Washington Square, sponsored by Air Force Aeronautical Research Laboratory, Army Research Office, Curham Office of Naval Research, N.Y.U., pp. 197-225 (1961)).

Among preferred luminescent label(s) for the multiple (e.g., dual) analyte assay with a biacridinium derivative are acridinium derivatives such as acridinium amides, dimethyl acridinium ester, metallic tetrapyrroles and nonmetallic tetrapyrrole but several other luminescent labels previously discussed are also suitable. A preferred nonmetallic tetrapyrrole is known by the acronym DPIX (deuteroporphyrin IX.2HCl or its free base). The preferred signal solution for producing emitted light by means of the DPIX (tetrapyrrole) label comprises at a pH from about 10.0 to about 14.0, trans, trans-5-(4-Nitrophenyl)-2,4-pentadienal, sodium di-2-ethylhexyl sulfosuccinate, the luminescent reactant luminol, glucose, benzyltrimethylammonium hydroxide, cumene hydroperoxide, sodium periodate, potassium superoxide and EDTA. The signal solution best suited for flashing the bound 10,10'-substituted-9,9'-biacridinium and acridinium derivatives comprises at a pH from about 10.0 to about 14.0 in 0.02 borax, EDTA, DMSO, D(-) fructose, potassium superoxide, 2-methyl-2-propanol and aqueous sodium tetraborate. If one of the analyte conjugates is an enzyme labeled analyte conjugate the substrate for tat enzyme can be included in the signal solution.

The symmetrical, uniformly symmetrical and asymmetrical biacridines disclosed herein also may be used as chemiluminescent signaling agents when using the conventional avidin-biotin or streptavidin-biotin interaction in assay or targeting systems. For example, a biacridine derivative may be covalently coupled to biotin to create a probe for the detection of avidin or streptavidin molecules or conjugates. The biacridine-biotin compound, for example, may be constructed as a bis-biotin derivative wherein both the 10 or 10' positions of the biacridine have been modified to possess one D-biotin substituent at each of the positions (e.g., a symmetrical molecule), or it may possess only a single biotin moiety off one of the 10 or 10' positions (e.g., an asymmetrical molecule). In either case, a biotinylated biacridine probe could be used to bind an antibody-streptavidin conjugate directed against an antigen of interest. Treatment of the resulting complex with the disclosed trigger solutions would then result in a chemiluminescent signal proportional to the amount of antigen present. Alternatively, a symmetrical, uniformly symmetrical or asymmetrical biacridine derivative may be used to modify avidin or streptavidin, creating a detection reagent useful for targeting biotinylated molecules. In this approach, a conjugate is then used to bind to an antigen target, and the streptavidin-biacridine conjugate is then used to detect bound antibody by binding to the biotinylated tag. Initiation of chemiluminescence by the triggering solution yields signals proportional to the amount of antigen present.

In addition, the invention is directed to a chemiluminescent homogeneous assay for detecting multiple (e.g., dual) analytes in a sample. In a competitive-type assay, the solid phase is coated with a binding partner specific for each different analyte. The solid phase may additionally be coated with a luminescent reactant in cases where a tetrapyrrole is used as one of the labels. The thus coated solid phase is then contacted with the sample, with a known amount of one of the analytes conjugated to a 10,10'-substituted-9,9'-biacridine label, with a known amount of the other analyte conjugated to luminescent label(s) other than the biacridine and with a polyion (such as poly-N-ethyl-4-vinylpyridinium bromide, poly-4-vinylpyrimidinium dichromate, polyvinylchloride, poly(vinylalcohol), or poly(vinylbenzyl chloride) capable of inhibiting unbound biacridine luminescent label conjugate such as anti-TSH-10,10'-substituted-9,9'-biacridine by preventing the overcoming of the oxidation potential of the luminescent label of the unbound conjugate (Vlasenko, S. B. et al, J. Biolum Chemilum, 4:164-176 (1989)). In an assay where it is necessary to inhibit unbound luminescent label antibody conjugate such as DPIX antibody conjugate a polycation can be used. Following contact, the solid phase is then treated with a signal solution capable of either producing emitted light by means of multiple (e.g., both) conjugates simultaneously or separately contacting the solid phase with a signal solution specific for one or more label(s) and measuring the emitted light and then separately contacting the solid phase with signal solution specific for emitting light by means of the label(s) of the other conjugate.

The invention is additionally directed to a chemiluminescent signal solution which comprises at a pH ranging from about 10.0 to about 14.0 an aqueous solution of about 150 mM to about 850 mM-potassium superoxide, preferably 250 to 500 mM in a buffer solution. The preferred buffer is sodium tetraborate but other solutions such as trizma base and boric acid also work well. The preferred luminescent molecules for use as labels in conjunction with this signal solution are the acridinium and 10,10'-substituted-9,9'-biacridine derivatives. However, isoluminol alone and deuteroporphyrin IX·2HCl (a commercially available compound, e.g., from Aldrich Chemical Co.) in conjunction with the luminescent reactant luminol when used with the KO₂ signal solution are also suitable labels. When the KO₂ in a signal solution is reacted with a luminescent molecule such as 10,10'-substituted-9,9'-biacridine or a derivative thereof or a luminescent label conjugate such as estradiol 17β-10,10'-substituted-9,9'-biacridine or anti-TSH- 10,10'-substituted-9,9'-biacridine, a signal to noise photon emission ratio of at least 20:1 at 1 ng/ml of label concentration is produced for at least 0.1 seconds. Depending upon the label the signal ratio can be 50:1, 100:1 or 200:1 and greater at 1 ng/ml of label concentration using this signal solution and a variety of biacridine labeled conjugates. The emission can also be manipulated to last from up to 6 seconds or longer.

The invention further describes a chemiluminescent signal solution which comprises at a pH from about 10.0 to about 14.0, 0.02 M aqueous borax, EDTA, DMSO, D(-) fructose, an oxidizing agent (such as potassium superoxide, perborates such as alkali metal perborates, peroxides such as hydrogen peroxide), and 2-methyl-2-propanol. This signal solution can trigger 10,10'-substituted-9,9'-biacridine derivative conjugate chemiluminescence producing a significant increase in light output and a change in light output kinetics from the output obtained with previously known signal solutions. When this solution is reacted with a luminescent label or a luminescent label conjugate such as 10,10'-substituted-9,9'-biacridine-antibody or anti-TSH-10,10'-substituted-9,9'-biacridine, a signal to noise photon emission ratio of at least about 500:1 at 1 ng/ml of label concentration may be produced for at least 0.1 seconds. Again, the signal ratio can be 50:1, 100:1 and even 700:1 and greater at 1 ng/ml of label concentration depending upon variations in the solution and the particular labeled conjugate. The emission can also be manipulated to last from up to 6 seconds or longer.

In addition, the invention is directed to a signal solution which comprises, at a pH ranging from about 10.0 to about 14.0, in an aqueous borax solution, EDTA, DMSO, D(-) fructose, at least one oxidizing agent, potassium superoxide and 2-Methyl-2-propanol. It is preferred in all aspects of the invention where this signal solution is used that it be prepared according to the procedure described in Example 2 with respect to components, sequence of component addition and component concentration. However, the components may be added in an altered sequence and other component concentrations which are also suitable are:
- Borax: 0.005-.05 M of aqueous buffer solution
- EDTA: 0.002-0.2 mM
- DMSO: 0-8 µl/ml of the above borax buffer solution
- D(-) fructose: 2-10 mg/ml of buffer solution
- Potassium Superoxide: 210-850 mM
- 2-Methyl-2-propanol: 0.05-0.25 ml/ml of buffer solution
When this solution is reacted with a luminescent label such as a 10,10'-substituted-9,9'-biacridine, the luminescent reactant may produce a signal to noise photon emission ratio of at least 500:1 at 1 ng/ml of luminescent label. Again, depending upon the label the signal ratio can be 50:1, 100:1, 200:1, etc. at 1 ng/ml of label concentration. The emission can also be manipulated to last from up to 6 seconds or longer.

The oxidant can be any added compound that is capable of overcoming the inherent oxidation potential of the 10,10'-substituted-9,9'-biacridine to create a flash or other emission of light. Potassium superoxide is preferred but other oxidants or combination of oxidants such as hydrogen peroxide, sodium perborate are alternatives. In PCT patent application (WO 96/00392) page 14, line 35 potassium superoxide was identified as preferred for overcoming the oxidation potential of the 10,10'-substituted-9,9'-biacridine. However, potassium superoxide in conjunction with other oxidants such as sodium perborate, osmium tetroxide or hydrogen peroxide is also another mixture capable of overcoming the inherent oxidation potential of the 10,10'-substituted-9,9'biacridine. These oxidants can also work alone to overcome the oxidation potential. Potassium superoxide is the preferred oxidant to produce light but it is not stable and is dangerous from a manufacturing standpoint.

The invention now being fully described, it will be apparent to one of ordinary skill in the art that many changes and modifications can be made thereto without departing from the spirit or scope of the appended claims.

### EXAMPLE 1

### Synthesis of 10,10'-Substituted-9,9'-Biacridine Derivatives

This approach to the synthesis of derivatized luminescent biacridine molecules included the synthesis of acridone by the cyclization of diphenylamine-2-carboxylic acids and N-benzoyl-diphenylamine-2-carboxylic acids as described by Acheson and Orgel, *supra*. (All chemicals and solvents can be obtained from Sigma/Aldrich, St. Louis, U.S.A. and Pacific Pac Inc., Hollister, CA). Acridone can also be purchased from Sigma/Aldrich.

In addition to the preparation of acridone, a methyl ester of a substituent molecule was synthesized for covalent attachment at the 10-nitrogen atom of acridone. A substituent molecule for derivatizing a biacridine molecule as used herein is a molecule having a functional group that provides for the further derivatization of the biacridine or for the attachment of the biacridine to other molecules such as antigens, antibodies, etc. Usually the substituent molecule used in the course of the invention has a molecular weight of about 10,000 or less. In this example, the methyl esterification of the substituent molecule alpha-bromo-para-toluic acid was carried out. Other molecules having good leaving group(s) (such as halogen atom(s)) at one end of the molecule and the presence of functional group(s) elsewhere on the molecule, can also serve as substituent molecules and be successfully esterified. A good example of another substituent molecule of this type is iodoacetic acid. Esterification was accomplished by reacting the substituent molecule in 10% boron trifluoride in methanol (25 ml of boron trifluoride-methanol per gram of substituent molecule was preferably added). This was allowed to react for at least 10 hours at room T° and the methyl ester was extracted with methylene chloride in a separation funnel. The extract was washed twice with H₂O, once with 0.1 M sodium bicarbonate and twice again with H₂O. The volume was reduced to dryness at 60°C on a rotavapor RE120 rotary evaporator. Alternate methods of methyl ester synthesis are the use of ether followed by the addition of diazomethane and the use of methanol containing 5% concentrated sulfuric acid.

The next step in the synthesis was the alkylation of acridone. To accomplish this, 5.4 mM of acridone and 6.5 mM of sodium hydride were added to 100 ml anhydrous tetrahydrofuran (THF). This mixture was then refluxed with stirring for 2 hours at 70°C under argon gas. To this mixture was added 5.5 mM of the substituent methyl ester (e.g., alpha-bromo-para-toluic acid methyl ester) and the combination was refluxed with stirring at 70°C for 10-13 hours. Silica gel thin layer chromatography (Baker Chemical Co., Phillipsburg, PA) at this time with 2% methanol in methylene chloride revealed a spot with R_{f} = 0.2 for hydrolyzed acridone- 10-substituent; a second spot at R_{f} = 0.4 for unreacted acridone; a third spot (very small) at R_{f} = 0.5 for unknown byproduct; a fourth spot at R_{f} = 0.6 for the acridone-10-substituent methyl ester (acridone-10-para-toluic acid methyl ester); and a fifth spot at R_{f} = 0.9 for unreacted substituent-methyl ester. The reaction mixture was a light lemon-brown color containing a precipitate (ppt.). The ppt. (containing mostly hydrolyzed acridone-10-substituent) was filtered off and discarded. The filtrate was extracted with ethyl acetate and water in a separation funnel to remove remaining salts and hydrolyzed material. The impurities remained in the aqueous phase. The volume of the organic phase (ethyl acetate phase) was reduced to dryness on a rotaevaporator at 60°C. Methylene chloride was then added and the unreacted (insoluble) acridone precipitate was filtered off. The methylene chloride extract was then eluted and purified on a silica-60 column with 3% ethyl acetate in methylene chloride. The acridone-10-para-toluic acid methyl ester was eluted and contained within the first yellow band. This material was again concentrated on a rotaevaporator with replacement of the ethyl acetate-methylene chloride eluant by methanol (through a continuous feed tube on the rotaevaporator). The purified acridone-10-substituent methyl ester precipitated as a light yellow crystalline material. This precipitate was filtered and washed with methanol (yield approximately 50%). These molecules and their acid precursors were active fluorophores with (for example) acridone-10-para-toluic acid and its NHS derivative exciting at 403 nm and emitting at 440 nm; and acridone-10-acetic acid and its NHS derivative exciting at 398 nm and emitting at 438 nm.

Acridone- 10-substituted intermediates (e.g., acridone- 10-acetic acid) were also directly synthesized by mixing well together 8.45 g of 2-chlorobenzoic acid, 7.80 g N-phenylglycine, 11.00 g anhydrous potassium carbonate and 0.30 g Cu++ powder in 6 ml H₂O. This mixture was then refluxed overnight over an oil bath at 160°C. Ethanol was added slowly and the product was dissolved in water, filtered and ppt. with HCl. The whole mixture was refiltered to remove unconsumed 2-chlorobenzoic acid and the remaining oil in that filtrate allowed to crystallize. The filtrate was dissolved in NaOH, filtered, acetic acid was added and the mixture was refiltered to remove further unreacted 2-chlorobenzoic acid. The product was precipitated by the addition of HCl (acid product) and dried. Then it was extracted with excess benzene and further purified by dissolving in sodium acetate solution, boiling with activated charcoal and reprecipitating with HCl. The pure product was again filtered and crystallized from dilute methanol to give a white ppt. (mp. 165-167°C).

Acridone-10-substituted molecules (e.g., acridone-10-acetic acid) were also synthesized, by refluxing a mixture of 500 mg (2.7 mM) acridone, 130 mg 80% NaH in mineral oil and 50 ml anhydrous Tetrahydrofuran (THF) under argon for 2-4 hours. Iodoacetic acid (540 mg, 2.7 mM) was then added and refluxing of the mixture was continued under argon for an additional 10 hours. The ppt. was filtered and the filtrate was purified on a reverse phase column under 20-30% ethanol elution. This purified material was then dried and taken up in THF and hydrolyzed with 4 N NaOH for 10 hours. Water was added and the mixture was filtered. The filter was washed with H₂O, and the mixture was brought to a pH of 8.0 with 1 N HCl. Final purification was performed on reverse phase silica gel column with 10 to 30% methanol in water. The volume was reduced on a rotaevaporator (e.g., RE120) and reprecipitation was carried out with 1 N HCl overnight at pH 2.5. The product was collected by centrifugation and washed with water once. It was dried on a lyophilizer (yield approximately 40%).

Conversion of the acridone-10-para-toluic-acid methyl ester to the quaternized 9-chloro-acridine-10-para-toluic acid methyl ester was accomplished by reacting the acridone-10-substituent methyl ester with phosphorous oxychloride (POCl₃). One milliliter of POCl₃ was added to each 50 mg of the purified methyl ester and this mixture was refluxed for 1 hour over an oil bath at 120°C.

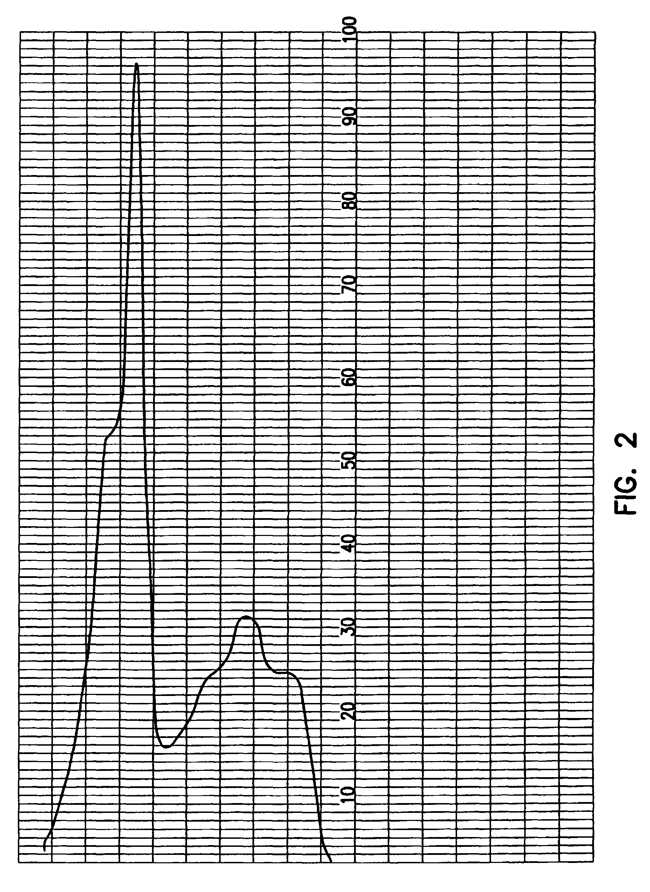

Dimerization and de-esterification of the 9-chloro-acridine-10-para-toluic acid methyl ester was accomplished by the addition of 1 g of cold zinc metal and 10 ml of freezing cold concentrated HCl/100 mg of 9-chloro-acridine-10-para-toluic acid methyl ester which was allowed to react under freezing conditions for anywhere from 1 to 10 hours. This reaction is violent and must be carried out under freezing conditions for 1-10 hours. The ppt. was then filtered off and washed with water. Purification of the acid filtrate was accomplished on a silica gel C-18 reverse phase column. The column was pretreated with methanol followed by 0.1 N nitric acid followed by 0.01 M phosphate buffer. Elution of the filtrate was first accomplished with methanol in 0.01 M phosphate buffer to remove the byproducts, unreacted materials and salts. The product, (10,10'-para-toluic acid-9,9'-biacridine salt, a disubstituted biacridine) which sticks to the top of the column, was then eluted with 30 to 90% methanol in 0.1 N nitric acid (the methanol strength should be increased from 30% to 90% to remove all product). Product eluted as a yellow band with approximately 50% methanol in 0.1 N nitric acid. The protonated purified dimer-dinitrate salt (10,10'-para-toluic acid-9,9'-biacridinium dinitrate) was then concentrated on a rotaevaporator at 60°C. to a small volume (5 ml) and was lyophilized to dryness. Scanning spectrophotometry on a Perkin-Elmer 552 Spectrophotometer revealed a characteristic absorbance with a preliminary shoulder at 460 nm, a first peak at 435 nm, a second shoulder at 415 nm, a major peak at 370 nm and a trailing shoulder at 355 nm (see Figure 2). NMR plot on a Bruker ARX 400 instrument (Rheinstetten-FO, Germany) gave a peak at 6.8 ppm indicating the presence of the methylene carbon attached to the 10 position nitrogen on the dimer and the presence of multiple aromatic carbon peaks in the 7 to 9 ppm range.

Further derivatization of the dimer with N-hydroxysuccinimide (NHS) was accomplished by adding (with stirring) 211 micromoles (uM) of dicyclohexylcarbodiimide to 141 uM of the dimer in dry dimethylformamide (DMF) (0.5 ml/mg of dimer). To this was added 211 uM of N-Hydroxysuccinimide which was allowed to react at room T° for 10 hours. Urea precipitates which formed during this reaction were filtered off. This NHS-ester of the label is very stable in an amber vial (at least one year). On reverse phase TLC the major peak did not move on elation with 90% methanol in 0.01 M phosphate buffer (a yellow spot at the origin under long wavelength U.V. light), but does move with an R_{f} = 0.2 in 01 nitric acid/70% methanol (v/v).

Conjugation of the 10,10'-para-toluo-NHS-9,9'-biacridinium dinitrate derivative to antibody began with the addition of 100 microliters of the DMF solution of the derivative to 1 mg of the antibody in PBS at pH 7.4. In this example polyclonal antibody to the beta-chain of thyroid stimulating hormone was conjugated, however, any antibody, analyte, polymer or binding protein can be utilized. This mixture was allowed to react at room T° for 10 hours and then 54 microliters of a 1 mg/ml solution of d-L-lysine was added to the antibody-derivative mix and was allowed to react for an additional 3 hours. This step is necessary for occupying unreacted NHS sites an the luminescent derivative-antibody conjugate.

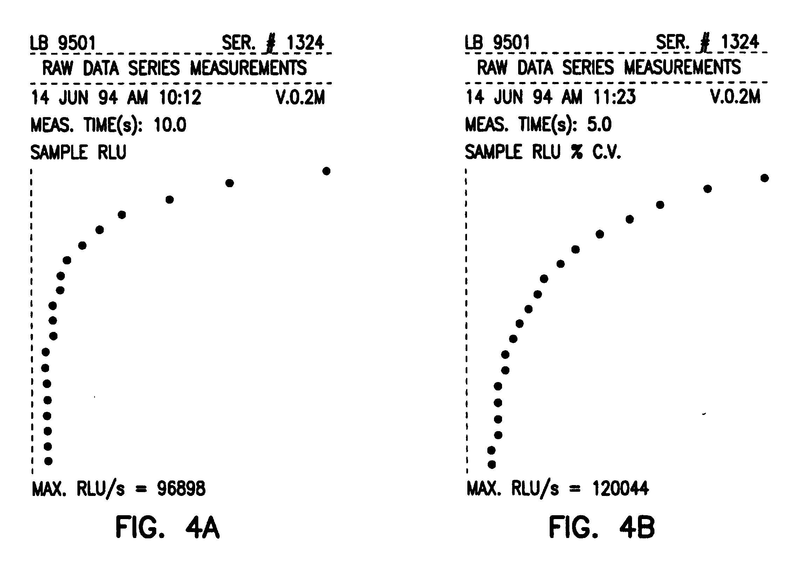

This antibody-10,10'-substituted-9,9'-biacridine conjugate was purified on a 20 cm Biogel P-10 column (BioRad; Hercules, CA) by eluting with a buffer containing 10 mM dibasic potassium phosphate and 0.1 M NaCl at a pH of 7.4. The antibody conjugate elated in the first fraction off the column which can be monitored by TLC and spectrophotometry. The antibody conjugate had two spectrophotometric peaks at 365 nm (small peak for the label) and at 275 nm for the antibody and was successfully flashed with the signal solution of the invention described in Example 2 resulting in very rapid light emission kinetics (see Figure 4A and 4B).

A mildly acidic environment (0.01 N HNO₃) stabilizes the labels and also gives the strongest signal-to-noise ratio. A wash solution containing 0.2 microliters Tween®-20/ml PSS brought to 0.01 N with HNO₃ should work well in separation-required assays. Exposing the label to 5 microliters of the final wash solution just before flashing may also enhance the signal.

### EXAMPLE 2

### Preparation of Signal Solution

The signal solution for the production of light from the new chemiluminescent molecules was formulated as follows:

To each 100 ml of 0.02 M sodium tetraborate was added the following with stirring:
a) 0.744 mg ((0.02 mM) ethylenediaminetetraacetic acid (EDTA)
b) 100 µl of dimethylsulfoxide (DMSO)
c) 400 mg (0.02 M) D(-) fructose
d) 1996 mg (280 mM) potassium superoxide (KO₂)
e) 17 ml 2-Methyl-2-propanol

### EXAMPLE 3

### Assay Comparing 10,10'-para-toluic acid-9,9'-Biacridinium Dinitrate and bis-N-Methylacridinium Dinitrate (Lucigenin)

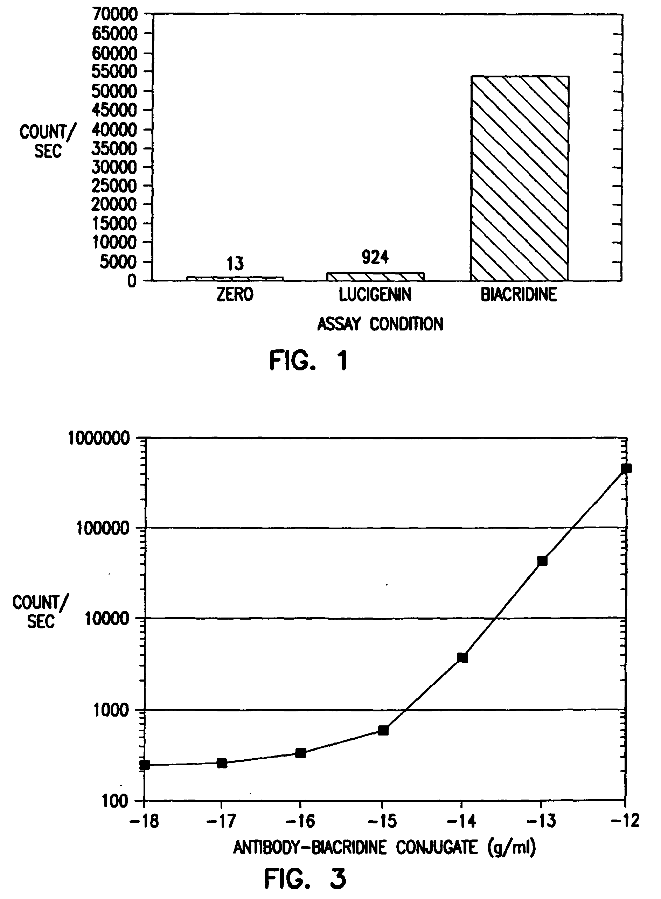

As shown in the bar graph of Fig. 1 showing the data of this Example 3, this example compared the signal obtained with signal solution only (Bar 1), a 1.9 nanomolar concentration of lucigenin in distilled H₂O (Bar 2), and a 1.3 nanomolar concentration of 10,10'-para-toluic acid-9,9'-biacridinium dinitrate in distilled H₂O (Bar 3). The signal solution was prepared according to Example 2, *infra*. The conditions for this assay were the addition of 300 microliters of signal solution to 5 µl of water in triplicate to 12 X 75 mm polystyrene tubes (VWR Scientific Inc., Philadelphia, PA) to obtain the zero signal. The signal of each chemiluminescent molecule was obtained by flashing 5 µl of each diluted luminescent label in triplicate in a Berthold Lumat luminometer.

### EXAMPLE 4

### Assay Demonstrating the Linearity of Signal with Increasing Dilutions of Anti-TSH-10,10'-para-toluo-9,9'-biacridine Conjugate

As shown in the line graph of Fig. 2 showing relative chemiluminescence of antibody conjugates, this example demonstrated the chemiluminescent functionality of the antibody conjugate and the linearity of signal with increasing dilutions. The anti-TSH-10,10'-para-toluo-9,9'-biacridine conjugate was dilated from 10⁻⁹ g/ml to 10⁻¹⁸ g/ml and 5 µl of each dilution was flashed with 200 µl of signal reagent in triplicate in a Berthold Lumat and the magnitude of the signal was recorded. The results were as follows:
10⁻⁹ g/ml-12,000,000 counts/sec; 10⁻¹² g/ml-570,000 counts/sec; 10⁻¹³ g/ml-37, 119 counts/sec; 10⁻¹⁴ g/ml-3,510 counts/sec; 10⁻¹⁵ g/ml-556 counts/sec; 10⁻¹⁶ g/ml-304 counts/sec; 10⁻¹⁷ g /ml-240 counts/sec; 10⁻¹⁸ g/ml-229 counts/sec; 0.0 g/ml-102 counts/sec.

### Mixed Biacridine NHS ester

The N-hydroxysuccinimide (NHS) ester of the N-methyl-N-toluic acid biacridine has been prepared using standard methods (NHS, dicyclohexylcarbodiimide [DCC] coupling). Proton NMR confirms the product as that in structure XV.

### Experimental

N-methyl-N-Toluic Acid Biacridinium Dinitrate (XIV): 1 gram of the N-toluic acid substituted acridone (XII) and 1.5 g of N-methylacridone (XI) are reductively coupled by treatment with zinc metal and concentrated HCl to give the acridan (XIII) as a yellow solid. The solid is filtered and oxidized to the biacridiniun dinitrate using aqueous HNO₃ at reflux. Upon cooling, the biacridinium dinitrate precipitates from solution. The product is purified by crystallization and chromatography. The NMR spectrum shows the product to be the desired N-methyl-N-toluic acid substituted biacridine (XIV).

### N-methyl-N-Toluic Acid Biacridinium Dinitrate NHS Ester (XV):

A solution of acid (XIV) (∼0.1 g), N-hydroxysuccinimide (∼0.1 g) and DCC (∼0.5 ml) in dimethylacetamide (5 ml) was stirred at room temperature overnight. The reaction was quenched wit 2 ml of acetic acid, diluted with 50 ml of acetonitrile and filtered to remove precipitated DCU (dicyclohexylurea). Purification by column chromatography gave the final product in acceptable purity. The final product contains residual NHS and a trace of the bis-toluic acid biacridine according to NMR analysis.

### Compounds Including Substitution on the Toluic Acid Ring, Substitution on the Acridine Ring and Asymmetrical Biacridine.

### Substitution on the Toluic Acid Ring

a. Fluoro Substituted
   *1). 10,10* 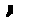 *-Bis[(4-carboxy-2-fluorophenyl)methyl]-9,9* 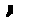 *biacridinium dinitrate* (II) and the corresponding NHS ester (III)
b. Methoxy Substituted
   *2). 10,10* 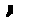 *-Bis [4-carboxy-2-methoxyphenyl)methyl]-9,9* 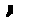 *biacridinium dinitrate* (IV) and corresponding NHS ester (V)

Several structural variations of 10,10'-bis[(4-carboxyphenyl)methyl]-9,9'-biacridinium dinitrate were prepared using similar synthetic techniques. These compounds contained substituents on the toluic acid appendage or on the biacridinium core. *N*-Hydroxysuccinimide (NHS) esters were prepared for the two derivatives with substituents on the toluic acid appendage.

The substituted-toluic acid biacridinium compounds are prepared by the alkylation of 9(10*H*)-acridone with a methyl 4-(bromomethyl)-3-substituted-benzoate. The methyl 4-(bromomethyl)-3-substituted-benzoates are commercially available (3-methoxy), or readily available using literature procedures (3-fluoro). The methyl esters of the alkylated acridones are saponified with methanolic sodium hydroxide to afford the respective sodium salts of the acids. These salts are dimerized using zinc and concentrated hydrochloric acid, and the intermediate dimers are oxidized with dilute nitric acid to give the substituted-toluic acid biacridinium compounds. The NHS esters of these acids are obtained by coupling the acid with NHS in the presence of *N*,*N'*-dicyclohexylcarbodiimide.

### Preparation of 10,10'-Bis[(4-N-succinimidyloxycarbonyl-2-fluorophenyl)methyl]-9,9'-biacridinium dinitrate (III):

**Methyl 3-fluoro-4-methylbenzoate.** A mixture of 3-fluoro-4-methylbenzoic acid (9.94 g, 61.3 mmol) and methanol (60 mL) was treated with a solution of 95% sulfuric acid (5.72 g, 55.4 mmol) in methanol (40 mL). The reaction mixture was heated at reflux for 1.5 hours. The mixture was cooled, poured into 100 mL of water, and extracted with ethyl ether (3x50 mL). A small amount of solid sodium chloride was added to facilitate phase separation. Evaporation of the solvent on a rotary evaporator gave a very pale yellow liquid (9.66 g, 94%).

**Methyl 4-(bromomethyl)-3-fluorobenzoate**. To a stirred mixture of *N*-bromosuccinimide (6.75 g, 37.5 mmol) in carbon tetrachloride (100 mL) was added a solution of methyl 3-fluoro-4-methylbenzoate (6.31 g, 37.5 mmol) in carbon tetrachloride (50 mL) and 2,2'-azobisisobutyronitrile [AIBN] (32 mg, 0.2 mmol). The reaction mixture was heated at 75 °C for 3 hours. To the flask was added additional AIBN (31 mg, 0.2 mmol) and the mixture was heated at reflux (∼80 °C) for 2.5 hours. The solids were filtered and rinsed with carbon tetrachloride. The filtrate was washed with 10% sodium thiosulfate (2x15 mL), saturated sodium bicarbonate (15 mL), and dried over sodium sulfate. Evaporation of the solvent on a rotary evaporator, followed by evacuation under high vacuum, gave a pale yellow oil (8.67 g). This liquid was purified by flash chromatography on a silica gel column eluted with 95:5 hexanes:ethyl acetate to afford a very pale yellow liquid (4.70 g, 51%). (Where the term 'hexanes' is used, the term means that the solvent is a mixture of hexane isomers).

**10-[(4-Carboxy-2-fluorophenyl)methyl]-9(10*H*)-acridone sodium salt**. To a stirred suspension of sodium hydride (0.84 g, 21.0 mmol) in tetrahydrofuran (45 mL) and dimethyl sulfoxide (12 mL) was added 9(10*H*)-acridone (3.33 g, 16.9 mmol) in portions over 10 min. The mixture was stirred at ambient temperature for 0.5 hours. To the resulting green solution was added a solution of methyl 4-(bromomethyl)-3-fluorobenzoate (4.69 g, 18.6 mmol) in tetrahydrofuran (5 mL). The mixture was heated for 2 hours at 69 °C and cooled to ambient temperature. The mixture was treated with acetic acid (0.56 g, 9.3 mmol) and stirred for 15 min. The solvent was concentrated on a rotary evaporator. The concentrate was diluted with water, ethyl acetate, and hexanes (65 mL each). The mixture was swirled and the resulting solid was collected by suction filtration. The filter cake was rinsed with water and ethyl acetate (25 mL). The solid was dried to give a light yellow powder (5.25 g, 86%).

A portion of this powder (2.50 g, 6.9 mmol) was suspended in methanol (13 mL). The suspension was treated with a solution of sodium hydroxide (0.54 g, 13.1 mmol) in water (22 mL). The mixture was heated at reflux for 2.5 hours, cooled to ambient temperature, and then in an ice-water bath for 0.5 hours. The resulting solid was collected by suction filtration. The filter cake was rinsed with water, acetone, ethyl acetate, and hexanes (5 mL each). The yellow powder was stirred with acetone (19 mL) for 2.3 hours and the solid was collected by suction filtration. The filter cake was rinsed with acetone and dried to give a pale yellow powder (2.37 g, 93%).

**10,10'-Bis [(4-carboxy-2-fluorophenyl)methyl]-9,9'-biacridinium dinitrate (II)**. To a stirred suspension of 10-[(4-carboxy-2-fluorophenyl)methyl]-9(10*H*)-acridone sodium salt (2.29 g, 6.20 mmol) in acetone (52 mL) was added zinc dust (6.79 g, 103 mmol). The mixture was stirred for 15 min at ambient temperature. The mixture was cooled to 12 °C in a cold water bath and 37% hydrochloric acid (61.15 g, 621 mmol) was added dropwise over 4 hours, maintaining the reaction temperature at 20±2 °C during the addition. After stirring for 1 hour (h) at ambient temperature, some zinc remained in the reaction mixture. Additional 37% HCl was added in portions (1.28 g, 13 mmol, 1.47 g, 15 mmol; 2.94 g, 30 mmol) at 0.5 hour intervals. The zinc was consumed after an additional 1.5 hours and the mixture was stirred overnight. The bright yellow solid was collected by suction filtration and rinsed thoroughly with water. The filter cake was rinsed with acetone, ethyl acetate, and hexanes (5 mL each), and dried briefly. The solid was suspended in acetone and treated with 6% nitric acid (55.50 g, 52.8 mmol). The mixture was heated at 70 °C for 2 hours, the color turning from yellow to light orange. Upon cooling, the solid was collected by suction filtration and rinsed with water. The filter cake was rinsed with acetone, ethyl acetate, and hexanes (5 mL each) and dried. The solid was further dried on a rotary evaporator at 50°C for 1.5 hours to afford a light orange powder (2.30 g, 94%).

**10,10'-Bis[(4-*N*-succinimidyloxycarbonyl-2-fluorophenyl)methyl]-9,9'-biacridinium dinitrate (III)**. A solution of 10,10'-bis[(4-carboxy-2-fluorophenyl)methyl]-9,9'-biacridinium dinitrate (II) (520 mg, 0.66 mmol) in *N*,*N*-dimethylformamide (DMF, 12 mL) was treated with *N*-hydroxysuccinimide (NHS, 206 mg, 1.79 mmol) and *N*,*N'*-dicyclohexylcarbodiimide (DCC, 310 mg, 1.50 mmol). The solids were rinsed in with DMF (4 mL). The mixture was stirred at ambient temperature for 20.5 hours. To the reaction was added NHS (24 mg, 0.21 mmol) and DCC (58 mg, 0.49 mmol) and stirring was continued for 5.75 hours. The reaction mixture was diluted with acetonitrile (8 mL) and stirred for 25 min. The solids were removed by suction filtration and rinsed thoroughly with acetonitrile. The filtrate was concentrated on a rotary evaporator and the concentrate was poured slowly into rapidly stirred ethyl acetate (100 mL). The resulting yellow solid was stirred for 15 min. and collected by suction filtration. The filter cake was rinsed with ethyl acetate and hexanes and dried to give a yellow powder (0.43 g, 66%).

### Preparation of 10,10'-Bis[(4-N-succinimidyloxycarbonyl-2-rnethoxyphenyl)methyl]-9,9'-biacridinium dinitrate (V):

**10-[(4-Carboxy-2-methoxyphenyl)methyl]-9(10*H*)-acridone sodium salt**. To a stirred suspension of sodium hydride (1.50 g, 37.5 mmol) in tetrahydrofuran (80 mL) and dimethyl sulfoxide (20 mL) was added 9(10*H*)-acridone (5.92 g, 30.0 mmol) in portions over 20 min. The mixture was stirred at ambient temperature for 0.5 hours. To the resulting green solution was added methyl 4-(bromomethyl)-3-methoxybenzoate (8.33 g, 33.0 mmol) as a solid in one portion. The mixture was heated for 4.75 hours at 65―70 °C and cooled to ambient temperature. To the flask was added sodium hydride (0.15 g, 3.75 mmol) and methyl 4-(bromomethyl)-3-methoxybenzoate (0.40 g, 1.05 mmol). The reaction was heated at 65-70 °C for 4 hours and cooled to ambient temperature. The mixture was treated with acetic acid (0.93 g, 15.5 mmol) and stirred for 0.5 hours. The reaction mixture was partitioned between water, ethyl acetate, and hexanes (115 mL each). A solid crystallized upon standing. The resulting solid was collected by suction filtration and rinsed with water and ethyl acetate. Additional solid separated in the filtrate and the filtrate was re-filtered twice. The organic layer of the filtrate was evaporated on a rotary evaporator to give a moist, olive-green solid (9.58 g). The filter cake was dried to give a light yellow powder (4.97 g). These two solids were combined with ethyl acetate (12 mL) and hexanes (108 mL). The solid was collected by suction filtration. The filter cake was rinsed with hexanes and dried to afford a light yellow-green powder (11.23 g, 100%).

A portion of this powder (5.46 g, 14.6 mmol) was suspended in methanol (27 mL) and water (10 mL). The mixture was heated for 2.6 hours at 75―80 °C, cooled to ambient temperature, and then cooled in an ice-water bath for 2 hours. The resulting solid was collected by suction filtration. The filter cake was rinsed with ice-cold water, acetone, ethyl acetate, and hexanes (10 mL each). The yellow powder was stirred with acetone (37 mL) for 1hourand the solid was collected by suction filtration. The filter cake was rinsed with acetone and dried to give a light yellow powder (4.70 g, 84%).

**10,10'-Bis[(4-carboxy-2-methoxyphenyl)methyl]-9,9'-biacridinium dinitrate (IV)**. To a stirred suspension of 10-[(4-carboxy-2-methoxyphenyl)methyl]-9(10*H*)-acridone sodium salt (2.29 g, 6.00 mmol) in acetone (50 mL) was added zinc dust (13.05 g, 198 mmol). The mixture was stirred for 15 min at ambient temperature. The mixture was cooled to 12―13 °C in a cold water bath and 37% hydrochloric acid (119.42 g, 1212 mmol) was added dropwise over 6 hours, maintaining the reaction temperature at 20±2 °C during the addition. The mixture was stirred overnight at ambient temperature. The bright yellow solid was collected by suction filtration and rinsed thoroughly with water. The filter cake was rinsed with acetone, ethyl acetate, and hexanes (5 mL each), and dried briefly. The solid was treated with 6% nitric acid (155.68 g, 148.2 mmol). The mixture was heated for 2.3 hours at 70―80 °C, the color turning from yellow to light orange. Upon cooling, the solid was collected by suction filtration and rinsed with water. The filter cake was rinsed with acetone, ethyl acetate, and hexanes (5 mL each) and dried. The solid was further dried on a rotary evaporator at 50 °C for 1.5 hours to afford a light orange powder (1.78 g, 73%).

**10,10' -Bis[(4-*N*-succinimidyloxycarbonyl)-2-methoxyphenylmethyl]-9,9'-biacridinium dinitrate (V)**. A solution of 10,10'-bis[(4-carboxy-2-methoxyphenyl)methyl]-9,9'-biacridinium dinitrate (IV) (536 mg, 0.66 mmol) in *N*,*N*-dimethylformamide (12 mL) was treated with *N*-hydroxysuccinimide (229 mg, 1.79 mmol) and *N*,*N'*-dicyclohexylcarbodiimide (328 mg, 1.59 mmol). The solids were rinsed in with DMF (4 mL). The mixture was stirred at ambient temperature for 21 hours. The reaction mixture was diluted with acetonitrile (8 mL) and stirred for 0.5 hours. The solids were removed by suction filtration and rinsed thoroughly with acetonitrile. The filtrate was concentrated on a rotary evaporator and the concentrate was poured slowly into rapidly stirred ethyl acetate (80 mL). The evaporation flask was rinsed with ethyl acetate (20 mL). The resulting yellow solid was stirred for 0.5 hours and hexanes (10 mL) was added. The solid was collected by suction filtration. The filter cake was rinsed with ethyl acetate and hexanes and dried to give a yellow powder (0.41 g, 62%).

### Substitution on the Acridine Ring

a. Fluoro Substituted.
   *1). 10,10* 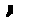 *-Bis[(4-carboxyphenyl)methyl]-3,3* 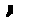 *-bis-(trifluoromethyl)-9,9* 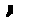 *-biacridinium dinitrate* (VI)
   *2). 10,10* 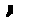 *-Bis[4-carboxyphenyl)methyl]-2,2* 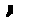 *-difluoro-9,9* 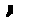 -*biacridinium dinitrate (VII)* and the corresponding NHS ester (VIII).
b. Methoxy Substituted
   *1). 10,10* 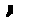 *-Bis[(4-carboxyphenyl)methyl]-2,2* 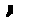 *-dimethoxy-9,9* 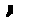 *-biacridinium dinitrate* (IX) and corresponding NHS ester (X).

### Fluorinated Biacridine Compounds

The acridine ring-substituted biacridinium derivative was prepared starting with *N*-[3-(trifluoromethyl)phenyl]anthranilic acid (Pellon, R.F., et al. *Tetrahedron Lett*. **1997**, 38, 5107-5110). The acid was cyclized to a mixture of 1- and 3-(trifluoromethyl)-9(10H)-acridones using polyphosphoric acid (Metz, G Synthesis. **1972**, 612-614). A pure sample of the 3-(trifluoromethyl)-9(10*H*)-acridone was isolated by flash chromatography. This acridone was alkylated with methyl 4-(bromomethyl)benzoate and the ester was saponified with methanolic sodium hydroxide. The resulting acid was dimerized with zinc and concentrated hydrochloric acid and the intermediate dimer was oxidized with dilute nitric acid to afford the trifluoromethyl-substituted biacridinium dinitrate.

An acridine ring-substituted biacridinium derivative was prepared starting with *N*-[4-fluorophenyl]anthranilic acid (Pellon, *supra*). The acid was cyclized to 4-fluoro-9(10*H*)-acridone using polyphosphoric acid (Metz, *supra*). This acridone was alkylated with methyl 4-(bromomethyl)benzoate and the ester was saponified with methanolic sodium hydroxide. The resulting acid was dimerized with zinc and concentrated hydrochloric acid and the intermediate dimer was oxidized with dilute nitric acid to afford the difluorobiacridinium dinitrate. The NHS ester of this acid is obtained by coupling the acid with NHS in the presence of *N*,*N'*-dicyclohexylcarbodiimide.

### Preparation of 10,10'-Bis[(4-carboxyphenyl)methyl]-3,3'-bis(trifluoromethyl)-9,9'-biacridinium dinitrate (VI):

### 3-(Trifluoromethyl)-9(10H)-acridone.

*N*-[3-(trifluoromethyl)phenyl]anthranilic acid (21.11 g, 0.075 mol), prepared as described by Pellón, was added in portions over 15 min to polyphosphoric acid (26.65 g) heated at 70―75 °C. The reaction temperature was raised to 120 °C and maintained for 2 hours. The mixture was cooled to ∼80 °C and water (53.02 g) was added slowly to produce a solid. The mixture was stirred at ambient temperature overnight. The solid was collected and rinsed with water. The moist green solid (51.11 g) was boiled for 5 min with sodium carbonate (11.22 g, 0.106 mol) in water (150 mL). The solid was collected while hot and the filter cake was rinsed with water. The solid was partially dried with suction. The light green solid was dried further in a vacuum oven for 28 hours at 50 °C to afford a light yellow-green powder (18.83 g, 95%) that was an approximately equal mixture of 1- and 3-(trifluoromethyl)-9(10*H*)-acridones. A portion (3.00 g) of the solid was purified by flash chromatography on a silica gel column eluted with 60:40 hexanes:ethyl acetate to give 3-(trifluoromethyl)-9(10*H*)-acridone as a yellow solid (0.15 g).

**10-[(4-Carboxyphenyl)methyl]-3-(trifluoromethyl)-9(10*H*)-acridone sodium salt.** To a stirred suspension of sodium hydride (23 mg, 0.58 mmol) in tetrahydrofuran (4 mL) and dimethyl sulfoxide (1 mL) was added 3-(trifluoromethyl)-9(10*H*)-acridone (120 mg, 0.46 mmol) in portions over 5 min. The mixture was stirred at ambient temperature for 0.5 hours. To the resulting green solution was added methyl 4-(bromomethyl)benzoate (122 mg, 0.52 mmol). The mixture was heated for 4.25 hours at reflux and cooled to ambient temperature. The mixture was treated with acetic acid (2 drops) and stirred overnight. The solvent was concentrated on a rotary evaporator. The concentrate was diluted with water, ethyl acetate, and hexanes (6 mL each). The mixture was swirled and the resulting solid was collected by suction filtration. The filter cake was rinsed with water, dried briefly, and rinsed with hexanes. Additional solid that separated in the filtrate was collected as described for the first crop. The solids were dried and combined to give a light yellow powder (107 mg, 57%).

A portion of this powder (82 mg, 0.20 mmol) was suspended in methanol (0.80 g). The suspension was treated with an aqueous solution of 2.4% sodium hydroxide (0.67 g, 0.40 mmol). The mixture was heated at 65 °C for 1.5 hours, cooled to ambient temperature, and evaporated under a stream of nitrogen. Water (∼1 mL) was added and the mixture was acidified by the addition of a few drops of 37% hydrochloric acid. The resulting solid was collected by suction filtration. The filter cake was rinsed with water and dried to give a pale yellow powder (69 mg, 87%).

**10,10'-Bis[(4-carboxyphenyl)methyl]-3,3** 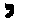 **-bis(trifluoromethyl) 9,9'-biacridinium dinitrate (VI)**. To a stirred suspension of 10-[(4-carboxyphenyl)methyl]-3-(trifluoromethyl)-9(10*H*)-acridone (50 mg, 0.126 mmol) in acetone (0.80 g) was added zinc dust (142 mg, 2.15 mmol). The mixture was stirred for 5―10 min at ambient temperature. The mixture was treated with 37% hydrochloric acid (1.29 g, 13.1 mmol) cooled to 37°C, added over 1 hour and the mixture was stirred overnight. The bright yellow solid was collected by suction filtration and rinsed thoroughly with water. The filter cake was rinsed with acetone (a few drops), and dried briefly. The solid was treated with 6% nitric acid (2.18 g, 2.08 mmol) at 70 °C for 1.3 hours. Additional 6% nitric acid (1.09 g, 1.04 mmol) was added and heating continued for 1.4 hours. Upon cooling, the solid was collected by suction filtration and rinsed with a small amount of water. The filter cake was dried to afford a light orange powder (46 mg, 82%).

### Preparation of 10,10'-Bis[(4-N-succinimidyloxycarbonylphenyl)methyl]-2,2'-difluoro-9,9'-biacridinium dinitrate (VIII):

***N*****-(2-Fluorophenyl)anthranilic acid**. A stirred suspension of copper powder (0.58 g, 0.009 mol) and potassium carbonate (8.46 g, 0.060 mol) in *N*,*N*-dimethylformamide was treated with 2-chlorobenzoic acid (19.17 g, 0.120 mol) and 4-fluoroaniline (27.10 g, 0.241 mol). The mixture was boiled for 2 hours. The cooled reaction mixture was poured into 300 mL of 1:1 water:37% hydrochloric acid. The solids were collected by suction filtration and washed with water. The purple solid was dissolved in sodium carbonate (11.47 g) in water (460 mL) and treated with activated carbon (11.48 g). The mixture was boiled for 5 min and filtered while hot. The cooled filtrate was acidified to pH 3 with 37% HCl. The resulting solid was collected by suction filtration and dried in a vacuum oven at 65 °C. A light yellow powder (20.57 g, 74%) was obtained.

**2-Fluoro-9(10*H*)-acridone**. *N*-(2-fluorophenyl)anthranilic acid (19.75 g, 0.085 mol) was added in portions over 15 min to polyphosphoric acid (30.18 g) heated at ∼65 °C. The reaction temperature was raised to 120 °C and maintained for 2 hours. The mixture was cooled to ∼80 °C and water (60 mL) was added slowly to produce a solid. The moist golden yellow solid was boiled for 5 min with sodium carbonate (12.62 g, 0.113 mol) in water (200 mL). The solid was collected while hot and the filter cake was rinsed with water. The solid was partially dried with suction. The solid was dried further in a vacuum oven at 60 °C to afford a light yellow-green powder (17.43 g, 96%).

**10-[(4-Carboxyphenyl)methyl]-2-fluoro-9(10*H*)-acridone sodium salt**. To a stirred suspension of sodium hydride (1.26g, 31.5 mmol) in tetrahydrofuran (67 mL) and dimethyl sulfoxide (16 mL) was added 2-fluoro-9(10*H*)-acridone (5.33 g, 25.0 mmol) in portions over 10 min. The mixture was stirred at ambient temperature for 0.5 hours. To the resulting dark orange solution was added methyl 4-(bromomethyl)benzoate (6.44 g, 28.1 mmol). The mixture was heated for 2.25 hours at 70 °C and cooled to ambient temperature. The mixture was treated with acetic acid (0.75 g, 12.5 mmol) and stirred for 0.5 hours. The solvent was concentrated on a rotary evaporator. The concentrate was shaken with water and ethyl acetate (100 mL each). Hexanes (100 mL) was added, the mixture shaken to mix, and allowed to stand overnight. The resulting solid was collected by suction filtration. The filter cake was rinsed with water, dried briefly, and rinsed with hexanes. The cake was dried to give a light yellow powder (7.45 g, 82%). A portion of this solid (3.61 g, 9.99 mmol) was suspended in methanol (19 mL). The suspension was treated with a solution of sodium hydroxide (0.77 g, 18.7 mmol) in water (31 mL). The mixture was heated at reflux for 2 hours, cooled to ambient temperature, and placed in an ice-water bath. The resulting solid was collected by suction filtration and the filter cake was rinsed with cold water. The moist solid was mixed with acetone (50 mL), filtered, and dried in a vacuum oven for 1.5 hours at 50 °C to give a light yellow powder (3.47 g, 100%).

**10,10'-Bis[(4-carboxyphenyl)methyl]-2,2'-difluoro-9,9'-biacridinium dinitrate (VII)**. To a stirred suspension of 10-[(4-carboxyphenyl)methyl]-2-fluoro-9(10*H*)-acridone sodium salt (2.08 g, 6.01 mmol) in acetone (50 mL) was added zinc dust (7.99 g, 121 mg atom). The mixture was stirred for 15 min at ambient temperature. The flask was cooled in a cold water bath and 37% hydrochloric acid (72.58 g, 737 mmol) was added dropwise over 3 hours, maintaining the reaction temperature at 20±2 °C during the addition. The zinc was consumed after stirring overnight The bright yellow solid was collected by suction filtration and rinsed thoroughly with water. The filter cake was rinsed with acetone, ethyl acetate, and hexanes (2 mL each), and dried briefly. The solid was suspended in acetone (10 mL) and treated with 6% nitric acid (252.89 g, 241 mmol). The mixture was heated for 2 hours at 70 °C, the color turning from yellow to olive to orange. Upon cooling, the solid was collected by suction filtration and rinsed with small amounts of water and 1:1 water:acetone. The solid was partially dried with suction and rinsed with ethyl acetate (50 mL) and hexanes. The solid was dried on a rotary evaporator at 45 °C for 1 hour to afford a light orange powder (1.65 g, 70%).

**10,10'-Bis[(4-*N*-succinimidyloxycarbonylphenyl)methyl]-2,2'-difluoro-9,9'-biacridinium dinitrate (VIII)**. A solution of 10,10'-bis[(4-carboxyphenyl) methyl]-2,2'-difluoro-9,9'-biacridinium dinitrate (VII) (521 mg, 0.66 mmol) in *N,N*-dimethylformamide (16 mL) was treated with *N*-hydroxysuccinimide (232 mg, 2.02 mmol) and *N,N'*- dicyclohexylcarbodiimide (333 mg, 1.60 mmol). The mixture was stirred at ambient temperature for 24 hours. The reaction mixture was diluted with acetonitrile (8 mL) and stirred for 0.5 hours. The solids were removed by suction filtration and rinsed thoroughly with acetonitrile. The filtrate was concentrated on a rotary evaporator and the concentrate was added dropwise into rapidly stirred 75:25 ethyl acetate:hexanes (85 mL). The concentrate was rinsed in with 75:25 ethyl acetate:hexanes (15 mL). The resulting yellow solid was stirred for 0.5 hours and the solid was collected by suction filtration. The filter cake was rinsed with 75:25 ethyl acetate:hexanes (100 mL), followed by pure hexanes, and dried to give a yellow powder (553 mg, 85%).

### Methoxy Substitution on Acridine Ring

An acridine ring-substituted biacridinium derivative was prepared starting with *N*-[4-methoxyphenyl]anthranilic acid (Pellon, *supra*). The acid was cyclized to 4-methoxy-9(10*H*)-acridone using polyphosphoric acid (Metz, *supra*). This acridone was alkylated with methyl 4-(bromomethyl)benzoate and the ester was saponified with methanolic sodium hydroxide. The resulting acid was dimerized with zinc and concentrated hydrochloric acid and the intermediate dimer was oxidized with dilute nitric acid to afford the dimethoxybiacridinium dinitrate. The NHS ester of this acid is obtained by coupling the acid with NHS in the presence of *N,N'*-dicyclohexylcarbodiimide.

### Preparation of 10,10'-Bis[(4-N-succinimidyloxycarbonylphenyl)methyl]-2,2'-dimethoxy-9,9'-biacridinium dinitrate (X):

**2-Methoxy-9(10*H*)-acridone**. *N*-(2-methoxyphenyl)anthranilic acid (19.70 g. 0.081 mol), prepared as described by Pellón. *supra*, was added in portions over 15 min to polyphosphoric acid (28.68 g) heated at ∼65 °C. The reaction temperature was raised to 110―115 °C and maintained for 2 hours. The mixture was cooled to ∼80 °C and water (60 mL) was added slowly to produce a solid. The mixture was allowed to stand at ambient temperature overnight. The solid was crushed with a spatula, stirred for 10 min, collected by suction filtration, and rinsed with water. The racist golden yellow solid was boiled for 5 min with sodium carbonate (12.02 g, 0.113 mol) in water (150 mL). The solid was collected while hot and the filter cake was rinsed with water. The solid was partially dried with suction. The solid was dried further in a vacuum oven for 22 hours at 60 °C to afford a yellow powder (17.69 g, 97%).

**10-[(4-Carboxyphenyl)methyl]-2-methoxy-9(10*H*)-acridone sodium salt**. To a stirred suspension of sodium hydride (1.25 g, 31.3 mmol) in tetrahydrofuran (67 mL) and dimethyl sulfoxide (16 mL) was added 2-methoxy-9(10*H*)-acridone (5.63 g, 25.0 mmol) in portions over 10 min. The mixture was stirred at ambient temperature for 0.5 hours. To the resulting dark green solution was added methyl 4-(bromomethyl)benzoate (6.43 g, 27.5 mmol). The mixture was heated for 1.25 hours at 70 °C and cooled to ambient temperature. The mixture was treated with acetic acid (0.75 g, 12.5 mmol), stirred for 0.5 hours, and allowed to stand overnight. The solvent was concentrated on a rotary evaporator. The concentrate was shaken with water and ethyl acetate (100 mL each). Hexanes (100 mL) was added, the mixture shaken to mix, and allowed to stand overnight. The resulting solid was collected by suction filtration. The filter cake was rinsed with water, dried briefly, and rinsed with hexanes. The cake was dried to give a yellow, crystalline solid (7.29 g, 78%).

A portion of this solid (3.61 g, 9.66 mmol) was suspended in methanol (19 mL). The suspension was treated with a solution of sodium hydroxide (0.80 g, 19.4 mmol) in water (31 mL). The mixture was heated at reflux for 2 hours, cooled to ambient temperature, and placed in an ice-water bath. The resulting solid was collected by suction filtration and the filter cake was rinsed with cold water. The moist solid was mixed with acetone, filtered, and dried to give a yellow powder (3.55 g, 96%).

**10,10'-Bis[(4-carboxyphenyl)methyl]-2,2'-dimethoxy-9,9'-biacridinium dinitrate (IX)**. To a stirred suspension of 10-[(4-carboxyphenyl)methyl]-2-methoxy-9(10*H*)-acridone sodium salt (2.29 g, 6.00 mmol) in acetone (50 mL) was added zinc dust (7.92 g, 120 mg atom). The mixture was stirred for 25 min at ambient temperature. The flask was cooled in a cold water bath and 37% hydrochloric acid (72.94 g, 740 mmol) was added dropwise aver 3 hours, maintaining the reaction temperature at 20±2 °C during the addition. The zinc was consumed after stirring overnight. The bright yellow solid was collected by suction filtration and rinsed thoroughly with water. The filter cake was rinsed with acetone, ethyl acetate, and hexanes (2 mL each), and dried briefly. The solid was suspended in acetone (5 mL) and treated with 6% nitric acid (158.39 g, 151 mmol). The mixture was heated for 0.5 hours at 40―45 °C and 0.5 hours at 55―60 °C, the color turning from yellow to olive to vivid orange. Upon cooling, the solid was collected by suction filtration and rinsed with small amounts of water, 1:1 water:acetone, ethyl acetate, and hexanes. The solid was further dried in a vacuum oven at 45 °C for 4hoursto afford a deep orange powder (1.69 g, 70%).

**10,10'-Bis[(4-*N*-succinimidyloxycarbonylphenyl)methyl]-2,2'-dimethoxy-9,9'-biacridinium dinitrate (X)**. A solution of 10,10'-bis[(4-carboxyphenyl)methyl]-2,2'-dimethoxy-9,9'-biacridinium dinitrate (IX) (535 mg, 0.66 mmol) in *N,N*-dimethylformamide (16 mL) was treated with *N*-hydroxysuccinimide (232 mg, 2.02 mmol) and *N,N'*-dicyclohexylcarbodiimide (331 mg, 1.60 mmol). The mixture was stirred at ambient temperature for 24 hours. The reaction mixture was diluted with acetonitrile (8 mL) and stirred for 0.5 hours. The solids were removed by suction filtration and rinsed thoroughly with acetonitrile. The filtrate was concentrated on a rotary evaporator and the concentrate was added dropwise into rapidly stirred 75:25 ethyl acetate:hexanes (75 mL). The concentrate was rinsed in with 75:25 ethyl acetate:hexanes (25 mL). The resulting orange solid was stirred for 0.5 hours and the solid was collected by suction filtration. The filter cake was rinsed with 75:25 ethyl acetate:hexanes, followed by pure hexanes, and dried to give a deep orange powder (584 mg, 88%).

### Asymmetrical Subsitution

### N-methyl-N-toluic acid biacridinium dinitrate (XIV) and its NHS ester (XV)-

### A Mixed Methyl Product

The N-methyl-N-toluic acid mixed biacridinium dinitrate (XIV) is synthesized by reductive coupling (Zn, HCl) of a mixture of N-methyl acridone (XI) and N-toluic acid acridone (XII). Use of a 4-5 equiv. excess of N-methyl acridone minimizes (though does not eliminate) formation of the unwanted bis-toluic acid product The coupling chemistry gives a mixture of all three possible products; separation by analytical thin layer chromatography (TLC) clearly shows the bis-N-methyl product (lucigenin precursor) and the mono- and diacids. The intermediate biacridylidene (XIII) mixture is oxidized in nitric acid and the product biacridinium dinitrate precipitates from solution on cooling. The isolated solid contains a large amount of lucigenin. Purification is accomplished by recrystallization and column chromatography.

The 360 MHZ NMR spectrum of the mixed biacridinium dinitrate is consistent with structure (XIV); the NMR also shows some residual lucigenin in the sample.

Materials used : N-methyl acridone (Aldrich, 19,250-3), Acridone (Acros, 40025-0250), Methyl 4-(bromomethyl)-benzoate (Aldrich, 34,815-5), Zinc dust (Fisher Scientific).

A mixed dimerization procedure is utilized in the preparation of a mono-NHS ester biacridinium dinitrate (XVI). Coupling of 10-[(4-carboxyphenyl)methyl]-9(10*H*)-acridone sodium salt with an excess with 10-[(4-carbomethoxyphenyl)methyl]-9(10*H*)-acridone and subsequent oxidation with nitric acid gives a mixture of symmetrical and unsymmetrical biacridinium compounds. The desired unsymmetrical biacridinium compound is isolated by flash chromatography. The free acid is coupled with NHS and DCC to give the mono-NHS ester (XVII). Another simplified version of synthesis for asymetrical compounds according to the present invention is performed by the use of different acridone reagants (e.g., an acridone with an A substituent in the 10 position and an acridone with a B substituent in the 10 position which are reacted in bulk (e.g., a single pot reaction) so tat a mixture of 10,10'-AA-biacridine, 10,10'-BB-biacridine, and 10,10'AB-biacridine is provided. The batch reaction product may or may not then be separated into one, two or three of the biacridines, as by HPLC or other sepration methods, depending upon the physical properties and differentiability amongst the three reaction products.

### Preparation of 10-[(4-N-succinimidyloxycarbonylphenyl)methyl]-10'-[(4-methoxycarbonyl-phenyl)methyl]-9,9'-biacridinium dinitrate (XVII):

**10-[(4-carboxyphenyl)methyl]-10'-[(4-methoxycarbonylphenyl)-methyl]-9,9'-biacridinium dinitrate (XVI)**. To a stirred suspension of 10-[(4-carboxyphenyl)methyl]-9(10*H*)-acridone sodium salt (0.84 g, 2.45 mmol) and 10-[(4-methoxycarbonylphenyl)methyl]-9(10*H*)-acridone (3.48 g, 9.90 mmol) in acetone (100 mL) was added zinc dust (13.05 g, 198 mmol) The mixture was stirred for 15 min at ambient temperature. The mixture was cooled to 15 °C in a cold water bath and 37% hydrochloric acid (122.94 g, 1248 mmol) was added dropwise over 4.2 hours, maintaining the reaction temperature at 20±2 °C during the addition. The mixture was stirred overnight at ambient temperature. The bright yellow solid was collected by suction filtration and rinsed thoroughly with water. The filter cake was rinsed with acetone, ethyl acetate, and hexanes (5 mL each), and dried to afford a yellow powder (3.89 g). A portion (2.00 g) of this solid was treated with 6% nitric acid (160.97 g, 153.3 mmol). The mixture was heated for 2 hours at 70 °C, the color turning from yellow to light orange. Upon cooling to 15 °C for 0.5 hours, the solid was collected by suction filtration, rinsed with water, and dried. The solid was further dried on a rotary evaporator at 50 °C for 1 hour to give a light orange powder (2.19 g). A portion of the solid was purified by flash chromatography on a silica gel column elated with 85:15 acetonitrile:0.1 N nitric acid. The fractions containing the half-ester were evaporated to give a moist solid (0.27 g). This solid was combined with 6% nitric acid (13.51 g) and heated for 1.5 hours at 70 °C. The solid was collected by suction filtration, rinsed with a small amount of water, and dried. The product was obtained as an orange powder (111 mg).

**10-[4-(*N*-succinimidyloxycarbonylphenyl)methyl]-10'-[4-(methoxy-carbonylphenyl)methyl]-9,9'-biacridinium dinitrate (XVII)**. A solution of 10-[4-(carboxyl)phenylmethyl]-10'-[4-(methoxycarbonyl)phenylmethyl]-9,9'-biacridinium dinitrate (XVI) (99 mg, 0.13 mmol) in *N,N*-dimethylformamide (1.94 g) was treated with *N*-hydroxysuccinimide (21 mg, 0.18 mmol) and *N,N* 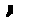 - dicyclohexylcarbodiimide (32 mg, 0.16 mmol). The mixture was stirred at ambient temperature for 16.5 h. To the mixture was added NHS (21 mg, 0.18 mmol) and stirring continued for 6 h. Another portion of NHS (21 mg, 0.18 mmol) and DCC (30 mg, 0.15 mmol) was added and stirring was continued for 27.5 h. Another portion of DCC (9 mg, 0.04 mmol) was added and stirring was continued for 4 h. The reaction mixture was diluted with acetonitrile (2 mL) and stirred for 1 h. The mixture was further diluted with acetonitrile (10 mL) and the solids were removed by suction filtration and rinsed thoroughly with acetonitrile. The filtrate was concentrated on a rotary evaporator and the concentrate was poured slowly into rapidly stirred ethyl acetate (100 mL). The resulting yellow solid was stirred overnight. The solid was collected by suction filtration. The filter cake was rinsed with ethyl acetate and hexanes and dried to give a yellow powder (250 mg).

### SECTION III: Analysis of Biacridinium Dinitrate Acids for Light Output

### Materials for Analysis of Biacridinium Dinitrate Labels

Phosphoric Acid; and ammonium chloride (Baker Chemical Co. Phillipsburg, PA)
Sodium phosphate, dibasic, anhydrous; glycine; sodium hydroxide; and Tris(hydroxymethyl) aminomethane hydrochloride (Tris HCl); maltose and potassium superoxide (Sigma, St. Louis, MO)
N, N-Dimethylformamide (99.9+%); and 2-methyl-2-propanol (Aldrich, Milwaukee, WI)
BupH™ Modified Dulbecco's PBS Packs (0.008 M sodium phosphate, 0.002 M potassium phosphate, 0.14 M sodium chloride and 0.01 M potassium chloride, pH 7.4); SuperBlock® Blocking Buffer in PBS (protein containing blocking solution in 0.01 M sodium phosphate, 150 mM sodium chloride and Kathon® an anti-microbial agent at pH 7.4) (Kathon is a registered trademark of Rohm and Haas), sequanal grade dimethylformamide (Dry DMF); Biotinylated Bovine Serum Albumin Coated White Microwell plate; Tween® 20 nonionic detergent (Tween®) is a registered trademark of ICI Americas); Streptavidin; Slide-A-Lyzer® Dialysis Cassette (U.S Patent No. 5,503,741); 18-gauge needle and 5 ml syringe (Pierce Chemical Company, Rockford, IL).

### Equipment

High Performance Liquid Chromatography (HPLC) System Hewlett-Packard Series 1100 Chem Station (Hewlett-Packard)
Gel Filtration Standards, HPLC Gel Filtration Column-Bio-Sil Sec-250 (300mm x 7.8mm); HPLC Guard Column-Bio-Sil Sec-250 (80mm x 7.8mm) (BioRad, Hercules, CA)
MLX Microtiter® Plate Luminometer (Dymex Technologies, Inc Chantily, VA)
Biacridinium dinitrate acids were dissolved in dry DMF and diluted in SuperBlock Blocking Buffer in PBS. The samples were added to a microwell plate at 10 mg/well. The plate was placed on the Dynex MLX Microtiter® Plate Luminometer. The trigger solution was injected into the wells of the plate at 100 ml/well. The wells were read 2 seconds/well to determine total relative light units (RLU). The results are shown in the following Table

### Analysis of Biacridinium Dintrate Acids that are Uniformly Symmetrically Subsituted on the Toluic Acid for Light Output

**TABLE 1**

| **Biacridinium Dinitrate Acid Substitution on Toluic Acid** | **Net Relative Light Unit at 10 µg/well** |
|---|---|
| 10,10 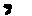 -para-toluic acid-9,9 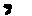 -biacridinium dinitrate (XVIII) | 610,669 |
| 10,10 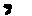 -Bis[(4-carboxy-2-fluorophenyl)methyl]-9,9 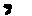 -biacridinium dinitrate (II) | 1,039,755 |
| 10 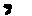 -Bis[(4-carboxy-2-methoxyphenyl)methyl]-9,9 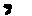 -biacridinium dinitrate (IV) | 290,596 |

### Asymmetrical Substitution on the N-position of Biacridinium Dinitrate

Acridine ring-substituted biacridinium salts were tested similarly to the other biacridines on a Laboratory Technologies ACCULYTE 10 Series Luminescence Counter. The sample was prepared in dry DMF and diluted in SuperBlock® Blocking Buffer in PBS. The biacridinium salts were added to a test tube at 10 pg/tube. The trigger solution was injected into the test tube 200 ml/tube. The tube was read at 2 seconds/tube to determine total RLU.

The results of this work are shown in Table 2 for the acridine rong substituted compounds, and in Table 3 for the unsymmetrical compounds..

**TABLE 2**

| Substitution an the Acridine Ring | |
|---|---|
| **Acridine Ring Substituted Biacridinium Dinitrate** | **Relative Light Unit at 10 pg/tube** |
| 10,10 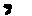 -Bis [(4-carboxyphenyl)methyl]-2,2 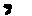 -dimethoxy-9,9 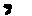 -biacridinium dinitrate (IX) | 579,078 |
| 10,10 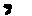 -Bis[4-carboxyphenyl)methyl]-2,2 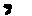 -difluoro-9,9 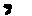 -biacridinium dinitrate (VII) | 481,611 |
| 10,10 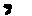 -Bis[(4-carboxyphenyl)methyl]-3,3 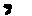 -bis(trifluoromethyl)-9,9 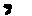 -biacridinium dinitrate(VI) | 71,683 |

**Table 3**

| Unsymmetrical | |
|---|---|
| **Biacridinium Dinitrate** | **Net Relative Light Unit at 10 µg/well** |
| N-Methyl-N toluic acid biacridinium dinitrate (XIV) | 527,656 |

### SECTION IV: Preparation of Biacridinium Conjugates

Streptavidin was dialyzed into 0.1 M Phosphate at pH 8.5 for a final concentration of 4 mg/ml. The biacridinium compounds were prepared in dry DMF and added to the streptavidin for a final 16:1 molar excess of biacridine to streptavidin. The N-hydroxysuccinimide reaction was allowed to react for 1 hour at room temperature. The reaction was stopped with glycine. To purify the conjugates, they were dialyzed in a Slide-a-Lyzer® Cassette into 0.1 M Tris HCl pH 6.0 (1 ml of conjugate/500 ml buffer) with three buffer changes. The antibody (streptavidin) conjugates had two spectrophotometric peaks using a Hitachi spectrophotometer at both 370 nm (biacridinium label) and 280 nm (streptavidin).

### SECTION V: Analysis by HPLC of Biacridinium Conjugates

HPLC Analysis of the conjugates was run with Dulbecco's Modified PBS pH 6.0 containing 10% DMF running buffer on a Gel Filtration Column (Bio-Sil® Sec-250 300 mm x 7.8 mm) in sequence. HPLC analysis results are shown in Tables 4 and 5 for toluic acid substituted and acridine ring substituted conjugates. Table 6 shows HPLC analysis of the asymmetric biacridine/Streptavidan conjugates.

**TABLE 4**

| Substitution on the Toluic Acid | | | | | |
|---|---|---|---|---|---|
| **HPLC Standard** | **MW** | **Retention Time at A280** | **Height (mAu)** | **Retention Time A370** | **Height (mAu)** |
| Thyroglobulin | 670,000 | 7.115 | 63.20991 | | |
| Bovine gamma globulin | 158,000 | 9.459 | 13.27857 | | |
| Chicken ovalbumin | 44,000 | 10.645 | 82.55029 | | |
| Equine myoglobin | 17,000 | 12.579 | 75.4627 | 12.582 | 123.8666 |
| Cyanocobalamin | 1,350 | 13.503 | 98.36756 | 13.503 | 163.5457 |
| | | | 138.795 | | |
| 10,10 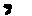 -para-Toluic acid-9,9 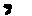 -biacridinium dinitrate labeled streptavidin | | 10.019 | 3.11764 | 10.035 | 3.68414 |
| | | 11.099 | 10.11713 | 11.093 | 12.56361 |
| 10,10 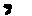 -Bis[(4-carboxy-2-fluorophenyl)methyl]-9,9 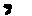 -biacridinium dinitrate labeled streptavidin | | 10.006 | 3.05856 | 10.029 | 25.6642 |
| | | 11.073 | 11.05375 | 11.06 | 74.3358 |
| 10,10 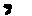 -Bis(4-carboxy-2-methoxyphenyl)methyl]-9,9 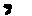 -biacridinium dinitrate labeled streptavidin | | 9.938 | 2.63377 | 9.926 | 2.88410 |
| | | 10.978 | 14.36486 | 10.97 | 16.45686 |

**TABLE 5**

| Substitution on the Acridine Ring | | | | | |
|---|---|---|---|---|---|
| **HPLC Standard** | **MW** | **Retention Time at A280** | **Height (mAu)** | **Retention Time A370** | **Height (mAu**) |
| Thyroglobulin | 670,000 | 6.994 | 92.46 | | |
| Bovine gamma globulin | 158,000 | 9.37 | 141.29 | | |
| Chicken ovalbumin | 44,000 | 10.551 | 137.73 | | |
| Equine myoglobin | 17,000 | 12.453 | 221.33 | | |
| Cyanocobalamin | 1,350 | 13.339 | 421.14 | | |
| | | | | | |
| 10,10 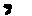 -para-Toluic acid-9,9 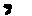 -biacridinium dinitrate labeled streptavidin | | 11.023 | 14.90 | 11.013 | 12.10 |
| | | 9.952 | 4.22 | 9.948 | 3.47 |
| 10,10 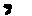 -Bis[(4-carboxyphenylmethyl-2,2 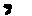 -dimethoxy-9,9 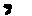 -biacridinium dinitrate labeled streptavidin | | 10.872 | 59.93 | 10.862 | 9.10 |
| 10,10 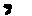 -Bis(4-carboxyphenyl)-methyl]-2,2 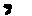 -difluoro-9,9 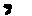 -biacridinium dinitrate labeled streptavidin | | 11.021 | 8.67 | 11.022 | 5.39 |
| | | | | | |
| ASYMMETRICAL RUN AT THE SAME TIME | | | | | |
| 10-[(4-carboxyphenyl) methyl]-10'-[(4-methoxycarbonylphenyl)-methyl]-9,9'-biacridinium dinitrate labeled streptavidin | | 11.383 | 63.33441 | 11.435 | 27.69574 |
| | | 10.286 | 8.08736 | 10.305 | 3.76629 |

**TABLE 6**

| **Asymmetrical** Biacridinium Dinitrate | | | | | |
|---|---|---|---|---|---|
| **HPLC Standard** | **MW** | **Retention Time at A280** | **Height (mAu)** | **Retention Time A370** | **Height (mAu)** |
| Thyroglobulin | 670,000 | 10.531 | 124.693 | | |
| Bovine gamma globulin | 158,000 | 13.909 | 153.384 | | |
| Chicken ovalbumin | 44,000 | 15.64 | 182.550 | | |
| Equine myoglobin | 17,000 | 18.447 | 223.585 | 18.451 | 276.793 |
| Cyanocobalamin | 1,350 | 19.865 | 345.100 | 19.865 | 447.105 |
| | | | | | |
| Streptavidin | | 14.719 | 2.78770 | | |
| | | 16.473 | 54.55226 | | |
| N-Methyl-N-Toluic Biacridinium dinitrate labeled streptavidin | | 14.965 | 11.6549 | 14.977 | 9.37322 |
| | | 16.568 | 72.85978 | 16.585 | 61.92606 |

### SECTION VI: Analysis by Functional Assay of Biacridinium Conjugates

Conjugates were prepared in SuperBlock® Blocking Buffer in PBS containing 0.05% Tween® 20 at 1 mg/ml. Samples were added to a biotinylated BSA coated white microwell plate and incubated for 1 hour at room temperature on a shaker platform. The wells of the microwell plate were washed with PBS containing 0.05% Tween-20. The plate was placed on a MLX Microtiter® Plate Luminometer set to inject 100 ml of the Trigger Solution/well. The wells were read 2 seconds/well to determine total integral RLU. The results are shown in Tables 7, 8 and 9.

**TABLE 7**

| **Functional Assay of Biacridinium-Streptavidin Conjugates Substituted on the Toluic Acid Ring** | |
|---|---|
| **Biacridinium Conjugated to Streptavidin** | **Net Relative Light Unit at 1 µg/ml** |
| 10,10 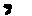 -para-Toluic acid-9,9 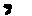 -biacridinium dinitrate | 61,298 |
| 10,10 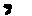 -Bis[(4-carboxy-2-fluorophenyl)methyl]-9,9 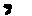 -biacridinium dinitrate | 58,432 |
| 10,10 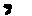 -Bis[(4-carboxy-2-methoxyphenyl)methyl]-9,9 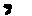 -biacridinium dinitrate | 61,006 |

**TABLE 8**

| **Functional Assay of Biacridine-Streptavidin Conjugates Substituted on the Biacridine Ring** | |
|---|---|
| **Biacridine Compound Conjugated to Streptavidin** | **Net Relative Light Unit at 1 µg/ml** |
| 10,10 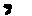 -para-Toluic acid-9,9 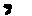 -biacridine (XVIII) | 22,932 |
| 10,10 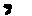 -Bis[(4-carboxyphenyl)methyl]-2,2 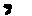 -dimethoxy-9,9 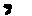 -biacridinium dinitrate (II) | 2,194 |
| 10,10 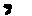 -Bis[(4-carboxyphenyl)methyl]-2,2 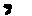 -difluoro-9,9 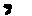 -biacridinium dinitrate (IV) | 17,413 |

**TABLE 9**

| **Functional Assay of Biacridinium-Streptavidin Conjugates Using Unsymmetrical Biacridinium Dinitrate** | |
|---|---|
| **Biacridinium Dinitrate Compound Conjugated to Streptavidin** | **Net Relative Light Unit at 1 µg/ml** |
| 10,10 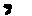 -para-Toluic acid-9,9 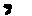 -biacridinium dinitrate (XVIII) | 911.11 |
| N-Methyl-N-Toluic Biacridinium dinitrate (IX) | 34.630.24 |
| 10-[(4-Carboxyphenyl)methyl]-10'-[(4-methoxycarbonylphenyl)methyl]-9,9'-biacridinium dinitrate labeled streptavidin (VII) | 18,143 |

### Section VIII

### Measurement of Multiple Luminescent Molecules In a Single Sample Using At Least One Signal Reagent

In this example it is demonstrated that at least one luminescent molecule can be triggered with at least one signal reagent and that at least one other luminescent molecule can be triggered by at least one other signal reagent, all in the same sample, and that light emitted from these luminescent molecules can be differentiated, detected and measured in a Berthold Lumat LB 9501 luminometer. Table 10 shows the relative total counts for various luminescent molecules when 5 microliters of each solution of the molecules are triggered separately with the signal reagents of this example. Clearly unique kinetic curves are generated by triggering with 5 microliters of chlorine solution (Porphyrin Products, Logan, UT) and 1 microliter of 10,10'-p-toluic acid-9,9'-biacridine together in the same test tube with a signal reagent of the invention (signal reagent 1: i.e. SR 1) containing 1.0 mg d-L-fructose, 0.13 ml 2-methyl-2-propanol and 440 mM KO₂ per mole of a 0.02 M aqueous solution of sodium tetraborate. The differences in kinetics were noted for each luminescent molecule as were the two different light peaks when both molecules were flashed in the same sample with a signal reagent of the invention.

### EXAMPLE 5

Four other (e.g., distinct) luminescent molecules are further used in this Example 5. Eight microliters of 4-(2-succinimidyloxycarbonylethyl)phenyl-10-methylacridinium-9-carboxylate trifluoromethane sulfonate-labeled DNA probe for the detection of **Neisseria gonorrhoeae** in 0.01 M lithium succinate (pH 5) and 0.1% (w/v) lithium lauryl sulfate (Gen Probe, Inc., San Diego, CA), 15 microliters of the 10,10'-acetic acid-9,9-biacridine-N-hydroxysuccinimide derivative in 0.01 M NaPhosphate, 5 microliters of deuteroporphyrin 1X2 HCl (Porphyrin Products, Logan, UT) in acetonitrile (Aldrich) and 5 microliters of hemin in water are contacted firstly with 200 microliters of a combination of 1.5% H₂O₂+0.1 N HNO₃ (signal reagent 2: i.e., SR 2) and, 0.5 sceonds later, with 200 microliters of 1 N NaOH (signal reagent 3: i.e., SR 3). The kinetic curves generated by each of these four luminescent molecules when separately triggered with SR 2+SR 3 were again recorded. Kinetic curves were generated by the SR 2+SR 3 separate triggering of 8 microliters of the acridinium ester conjugate and 15 microliters of the biacridine-NHS derivative (with the then simultaneous triggering of these two molecules of the example in the same sample). Following the triggering of these four luminescent molecules in the same sample with SR 1+SR 2, they were then separately triggered with 200 ul of a signal reagent containing 28 microliters of a 1% solution (w/v) of sodium di-2-ethylhexyl sulfosuccinate (AOT: K&K Laboratories, Cleveland, OH), 3.4 mg dextrose, 14 microliters of a 5-amino-2,3-dihydro-1,4-phthalazinedione (luminol) solution (880 microliters of 10 mM luminol in H₂O added to 100 ml of 0.1 M Trizma base in H₂O), and KO₂ to bring the pH to 12.86 per 1.0 ml of 0.1 M. SR. 4. Kinetic curves were generated by these four luminescent molecules together in the same sample when triggered with SR 2+SR 3 and then subsequently with SR 4. Luminol is the non-limiting luminescent reactant of this example, however, and many other luminescent reactants such as enzymes, dioxetanes, peroxyoxylates, isoluminols, ethylbenzene, hypoxanthines and isoxanthopterins can be utilized. In this example, light emitted from luminescent molecules triggered with the first signal reagent is differentiated, detected and measured and then, following triggering of the other luminescent molecules, that separate light emitted is differentiated, detected and measured (a Berthold Lumat LB 9501 luminometer was used in this example). Among the ways differentiation can be accomplished are through the use of differences in the kinetics of light output produced by each luminescent molecule and differences in the wavelengths of light produced by each luminescent molecule (e.g., the acridinium ester derivative of the example emits light at a peak wavelength of 435 lambda while the biacridine derivative of the example emits light at a peak wavelength of 495 lambda). In this example, the acridinium ester derivative molecules and biacridine derivative molecules are triggered with a first signal reagent and the tetrapyrrole molecules are separately triggered with a second signal reagent in the same tube. When these luminescent molecules are used as labels to measure analytes of interest, the amount of light differentiated, detected and measured is directly or indirectly proportional to the amounts of specific analytes of interest depending on the assay format and conditions.

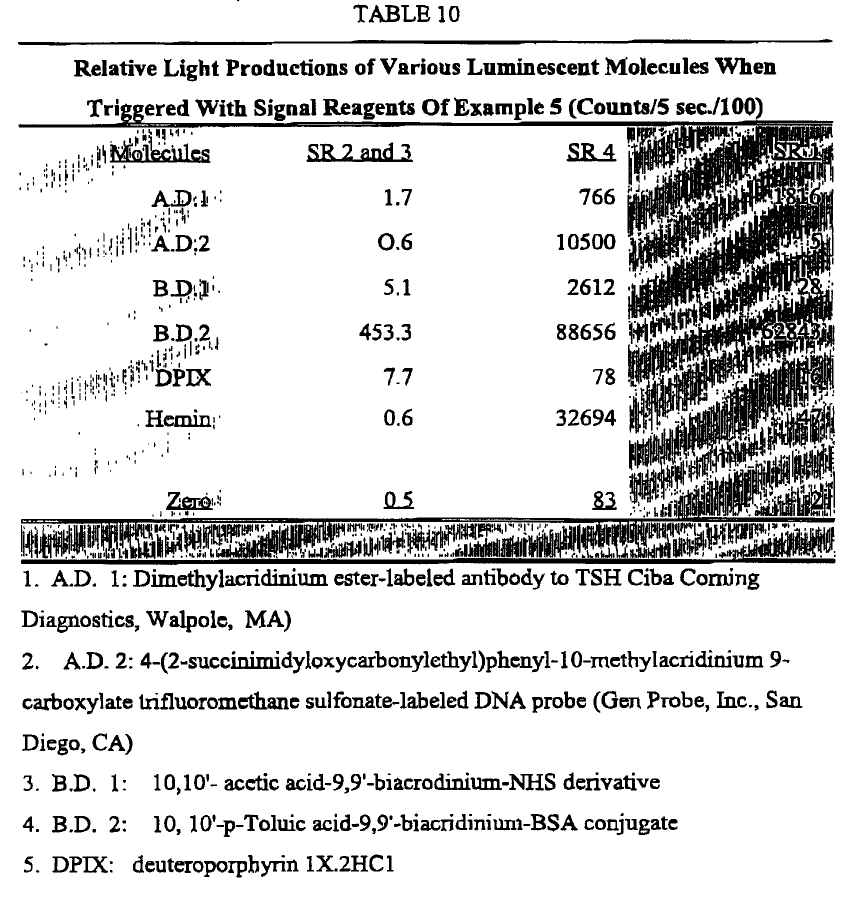

## Claims

1. A composition comprising a 10,10'-substituted-9,9'-biacridinium salt conjugated to an antigen, an antibody or a hapten wherein the substituent substituted at the 10 position is different from the substituent substituted at the 10'position.

2. The composition of claim 1 wherein at least one of said substituent at the 10 position and the substituent at the 10' position is an organic group.

3. The composition of claim 1 wherein said substituent at the 10 position and the substituent at the 10'position are different organic groups.

4. The composition of claim 1 wherein at least one of said 10-substituent and said 10'-substituent is linked to said 9,9'-biacridine through a para-toluo group.

5. The composition of claim 2 wherein only one of said 10-substituent and said 10'-substituent is linked to said 9,9'-biacridine a) through a para-toluo group that is derivatized with an N-hydroxysuccinimide group, sulfo-N-hydroxysuccinimide, polyfluorophenol activated ester or an acyl imidazole or b) directly to a succinimidyloxycarbonyl group, sulfo-N-hydroxysuccinimide, polyfluorophenol activated ester or an acyl imidazole.

6. The composition of claims 1, 2, 3, 4, or 5 wherein one of said 10-substituent and said 10'-substituent is an alkyl group.

7. An asymmetric 10,10'-substituted-9,9'-biacridine wherein a substituent substituted at the 10 position is different from a substituent substituted at the 10'position of the 9,9'-biacridine.

8. The asymmetric 10,10'-substituted-9,9'-biacridine of claim 7 wherein said substituent at the 10 position and the substituent at the 10'position are different organic groups.

9. The asymmetric 10,10'-substituted-9,9'-biacridine of claim 8 wherein at least one of said 10-substituent and said 10'-substituent is linked to said 9,9'-biacridine through a para-toluo group.

10. The asymmetric 10,10'-substituted-9,9'-biacridine of claim 8 wherein only one of said 10-substituent and said 10'-substituent is derivatized with an N-hydroxysuccinimide group, polyfluorophenol group, an imidazole activated ester or sulfo-N-hydroxysuccinimide group.

11. The asymmetric 10,10'-substituted-9,9'-biacridine of claim 7 wherein one of said 10-substituent and said 10'-substituent is an alkyl group.

12. A symmetric, uniformly symmetric or asymmetric 10,10'-substituted-9,9'-biacridine compound wherein at least one fused phenyl ring on said 9,9'-biacridine is substituted with a group other than hydrogen without the presence of amino or substituted amino groups on any fused phenyl ring.

13. The compound of claim 12 wherein a substituent present on said at least one fused phenyl ring on said 9,9'-biacridine is selected from the group consisting of alkyl groups, perfluoroalkyl groups, alkenyl groups and halogen.

14. The compound of Claim 12 which is symmetrical and at least one fused benzene ring on each of the two acridine moieties forming the 9,9'-biacridine is substituted with the same group without the presence of amino groups or substituted amino groups.

15. The compound of claim 12 wherein said 10,10'-substituted-9,9'-biacridine is a 10,10'-para-toluic acid-9,9'-biacridine, a 10,10'-para-toluo-9,9'-biacridine, a 10,10'-aceto-9,9'-biacridine or a 10,10'-acetic acid-9,9'-biacridine.

16. The symmetric, uniformly symmetric or asymmetric 10,10'-substituted-9,9'-biacridine compound of claim 12 wherein said group other than hydrogen is selected from the group consisting of alkyl group, aryl group, heterocyclic group, halogen, sulfonate, alkoxy, aryloxy, carboxyl, nitrile, and inorganic acids group.

17. The symmetric, uniformly symmetric or asymmetric 10,10'-substituted-9,9'-biacridine compound of claim 12 wherein each acridine has only one substituent other than hydrogen attached to rings thereof.

18. A chemiluminescent system for emitting measurable light useful in a chemical assay, an immunoassay, a ligand binding assay or a nucleotide assay, said system comprising: at a pH ranging from about 10.0 to about 14.0, a 10,10'-substituted-9,9'-biacridine compound of claim 12 which is symmetric, uniformly symmetric or asymmetric, and has an oxidation potential, a signal solution having an oxidant or a combination of oxidants capable of overcoming the oxidation potential of the symmetric, uniformly symmetric or asymmetric 10,10'-substituted-9,9'-biacridine, said symmetric, uniformly symmetric or asymmetric 10,10'-substituted-9,9'-biacridine being bound to an analyte, or to a binding partner of an analyte or to a ligand of a binding partner to an analyte.

19. A chemiluminescent system comprising the symmetric, uniformly symmetric or asymmetric 10,10'-substituted-9,9'-biacridine of claim 21 in a solution with a chelating agent a sulfoxide, a reducing sugar, an alcohol and an optional buffering agent.

20. The chemiluminescent system of claim 19 further comprising a combination of oxidants wherein the chelating agent is a polyamine acetic acid, the sulfoxide is dimethylsulfoxide, and the alcohol is 2-methyl-2-propanol.

21. The chemiluminescent system of claim 19 wherein said analyte is a nucleic acid, an antigen, an antibody, a hapten, a hapten conjugate, a macromolecule, a protein or a polymer.

22. The chemiluminescent system of claim 19 wherein said symmetric, uniformly symmetric or asymmetric 10,10'-substituted-9,9'-biacridine is bound to an analyte, a binding partner of the analyte or to a ligand of a binding partner of the analyte by means of a biotin-avidin or biotin-streptavidin bridge.

23. A method for using the symmetric, uniformly symmetric or asymmetric 10,10'-substituted-9,9'-biacridine of claim 12 in a chemiluminescent homogeneous assay for detecting the presence of or measuring the amount of an analyte in a sample comprising either:
A)
(a) providing a solid phase coated with a specific binding partner for said analyte;
(b) contacting said solid phase with said sample and with a predetermined amount of said symmetric, uniformly symmetric or asymmetric 10,10'-substituted-9,9'-biacridine as an analyte conjugate, said symmetric, uniformly symmetric or asymmetric 10,10'-substituted-9,9'-biacridine having an oxidation potential, and with a predetermined amount of a polyanion that prevents unbound symmetric, uniformly symmetric or asymmetric 10,10'-substituted-9,9'-biacridine-analyte conjugate from mediating luminescence, at least some of said binding partner binding to at least some of said symmetric, uniformly symmetric or asymmetric 10,10'-substituted-9,9'-biacridine-analyte conjugate;
(c) contacting the solid phase from (b) with signal solution comprising at a pH ranging from about 10.0 to about 14.0, oxidant that overcomes or a combination of oxidants that overcome the oxidation potential of the symmetric, uniformly symmetric or asymmetric 10,10'-substituted-9,9'-biacridine in the bound symmetric or asymmetric 10,10'-substituted-9,9'-biacridine analyte conjugate to emit light; and
(d) measuring the amount of light emitted in (c) wherein said amount of emitted light will be proportional to the amount of analyte present in said sample; or
B)
(a) providing a solid phase coated with a first specific binding partner and a second specific binding partner, said first binding partner being specific for said first analyte and said second binding partner being specific for said second analyte;
(b) contacting said solid phase with said sample and with a non-biacridine label-first analyte conjugate and a symmetric, uniformly symmetric or asymmetric 10,10'-substituted-9,9'-biacridine label-second analyte conjugate, at least some of said first analyte conjugate binding to at least some of said first binding partner and at least some of said second analyte conjugate binding to at least some of said second binding partner;
(c) separating unbound conjugates from bound conjugates by washing said contacted solid phase;
(d) contacting said washed solid phase in (c) with either a signal solution specific for said symmetric, uniformly symmetric or asymmetric 10,10'-substituted-9,9'-biacridine or a signal solution specific for said non-biacridine label to produce light by means of a chemical reaction;
(e) detecting or measuring said light from said reaction in (d);
(f) contacting the solid phase from (d) with a signal solution specific for said symmetric, uniformly symmetric or asymmetric 10,10'-substituted-9,9'-biacridine label, if the signal solution in (d) was a solution specific for said non-biacridine label, or with a signal solution specific for said non-biacridine label, if the solution in (d) was a solution specific for said symmetric, uniformly symmetric or asymmetric 10,10'-substituted-9,9'-biacridine label, to produce light by means of a chemical reaction;
(g) detecting or measuring said light from said reaction in (f); and
(h) detecting said first and said second analyte or determining the amount of said first or said second analyte from the light detected or measured in steps (e) and (g).

24. In a ligand binding assay method for determining the presence or measuring the concentration of an unknown amount of a bio-active analyte in a fluid sample whereby such presence or concentration is determined by using a label and a signal solution to produce a detectable or measurable reaction product, an improvement is set out comprising using a symmetric, uniformly symmetric or asymmetric 10,10'-substituted-9,9'-biacridine of claim 12 as the label and a mixture of EDTA, DMSO, D(-) fructose, KO₂ and 2-methyl-2-propanol in aqueous sodium tetraborate as the signal solution.

25. A composition according to claims 1 or 12 comprising a 10,10'-substituted-9,9'-biacridinium salt conjugated to an antigen, a hapten, an antibody, protein, carbohydrates, DNA, RNA, nucleosides, lipids, organic molecule, or organic polymer wherein the substituent substituted at the 10 position is different from the substituent substituted at the 10'position.

26. A method for using a 10,10'-substituted 9,9'-biacridine in a chemiluminescent heterogeneous assay for detecting, differentiating or measuring the presence of more than one luminescent molecule in a sample comprising:
(a) providing a solid phase coated with specific binding partners for said molecules, binding partners for said molecules bound to specific analytes, binding partners for said molecules bound to specific binding partners of said analytes or binding partners for said molecules bound to specific ligands of said binding partners of said analytes;
(b) contacting said solid phase with said sample, with said sample and said luminescent molecule specific analyte conjugates, said luminescent molecule specific binding partner conjugates of said analytes or said luminescent molecule specific ligand conjugates of said binding partners of said analytes, with at least one of said luminescent molecules being a 10,10'-substituted-9,9'-biacridine wherein said 10,10'-substituted-9,9'-biacridines are bound by covalent binding of at least one of said substituted groups at said 10 or 10' position of said 10,10'-substituted-9,9'-biacridines, the group substituted at the 10 or 10' position of said biacridines is not trisubstituted on said biacridines and the other luminescent molecules and the 10,10'-substituted-9,9'-biacridines are not the same molecule, at least some of said luminescent molecules and said luminescent molecule conjugates binding to at least some of said specific binding partners and at least some of said biacridine molecules and said biacridine molecule conjugates binding to at least some of said specific binding partners;
(c) separating unbound luminescent molecules and unbound conjugates from bound luminescent molecules and bound conjugates by washing said contacted solid phase;
(d) contacting the washed solid phase in (c) with signal reagent specific for at least one of said luminescent molecules, and with separate signal reagent specific for at least one other of said luminescent molecules to produce light by means of chemical reactions;
(e) detecting, differentiating or measuring said light from said reactions in (d); and
(f) detecting said luminescent molecules of said analytes or determining the amounts of said luminescent molecules and said analytes from the light detected, differentiated or measured in step (e).

27. A composition comprising a symmetric, uniformly symmetric or asymmetric 10,10'-substituted-9,9'-biacridinium salt conjugated to antigen, antibody, macromolecule, protein, nucleic arid, polymer, analyte, binding partner of analyte, ligand of binding partner to analyte, hapten conjugate or hapten wherein at least one substituent on the biacridinium is a reactive group.
